# EUROPEAN PATENT SPECIFICATION

(11) **EP 1 487 444 B1**
(45) Date of publication and mention of the grant of the patent: **30.12.2009**
(21) Application number: 03744849.5
(22) Date of filing: 24.03.2003
(51) Int. Cl.: A61K 31/44, A61K 31/45, A61K 31/495, A61K 31/4406, A61P 37/00, A61P 35/00, A61P 43/00

(54) **USE OF PYRIDYL AMIDES AS INHIBITORS OF ANGIOGENESIS**
VERWENDUNG VON PYRIDYLAMIDEN ALS ANGIOGENESEHEMMER
UTILISATION D'AMIDES PYRIDYLIQUES EN TANT QU'INHIBITEURS DE L'ANGIOGENESE

(30) Priority: 27.03.2002 EP 02006697
(43) Date of publication of application: 22.12.2004
(73) Proprietor: Astellas Deutschland GmbH, 80992 München (DE)
(72) Inventor: BIEDERMANN, Elfi, 2353 EW Leiderdorp (NL); LÖSER, Roland, 2353 EW Leiderdorp (NL); RATTEL, Benno, 2353 EW Leiderdorp (NL)
(74) Representative: HOFFMANN EITLE
(86) International application number: PCT/EP2003/003060
(87) International publication number: WO 2003/080054

(56) References cited:
- WO-A-98/02170
- DE-A- 19 624 659
- DE-A- 19 624 704
- DE-A- 19 756 212
- DE-A- 19 756 235
- DE-A- 19 756 236
- DE-A- 19 756 261
- US-A- 4 778 796
- NIE DAOTAI ET AL: "Eicosanoid regulation of angiogenesis: Role of endothelial arachidonate 12-lipoxygenase." BLOOD, vol. 95, no. 7, 1 April 2000 (2000-04-01), pages 2304-2311, XP002208812 ISSN: 0006-4971
- COLAVITTI RENATA ET AL: "Reactive oxygen species as downstream mediators of angiogenic signaling by vascular endothelial growth factor receptor-2/KDR." JOURNAL OF BIOLOGICAL CHEMISTRY, vol. 277, no. 5, February 2002 (2002-02), pages 3101-3108, XP001074657 February, 2002 ISSN: 0021-9258
- NISHIKAWA ET AL: "Acrylamide derivatives as antiallergic agents. 2. Synthesis and structure activity relationships of N-[4-[4-(diphenylmethyl)-1- piperazinyl]butyl]-3-(3-pyridyl)acrylamide s" JOURNAL OF MEDICINAL CHEMISTRY, AMERICAN CHEMICAL SOCIETY. WASHINGTON, US, vol. 32, no. 3, 1989, pages 583-593, XP002101517 ISSN: 0022-2623

## Description

### Field of the invention

The invention relates to the use of pyridyl amide derivatives according to the claims - alone or in combination with one or more other pharmaceutically active compounds - in the treatment of a disease according to the claims in mammals, especially in humans, and the use of such a compound - alone or in combination with one or more other pharmaceutically active compounds - in the manufacture of a pharmaceutical composition for the treatment according to the claims of mammals, especially of humans, in which inappropriate, excessive or undesirable angiogenesis has occurred, and to the prevention thereof.

### Background of the invention

Chronic diseases are often accompanied by profound angiogenesis, which can contribute to or maintain and an inflammatory and/or proliferative state, or which leads to tissue destruction through the invasive proliferation of blood vessels.

Angiogenesis is generally used to describe the development of new or replacement of blood vessels, or neovascularization. It is a necessary and physiologically normal process by which the vasculature is established in the embryo. Angiogenesis does not occur, in general, in the normal adult. Many diseases, however are characterized by persistent and unregulated angiogenesis. Examples thereof are proliferative retinopathies, rheumatoid arthritis and psoriasis (N. Ferrara, J. Mol. Med. (1999) 77: pp. 527-543).

Further, it has been shown that vascular endothelial growth factor (VEGF) is a fundamental regulator of normal and abnormal angiogenesis. VEGF appears to be essential for embryonic vasculogenesis and angiogenesis. It was established that e.g. the concentrations of VEGF are elevated in the aqueous and vitreous humors of patients with proliferative retinopathies such as the diabetic retinopathy (see: N. Ferrara, J. Mol. Med. (1999) 77: pp. 527-543; and references cited therein).

The recognition or the involvement of VEGF and/or angiogenesis (or in other words the growth of vessels) has been accompanied by research to identify and develop inhibitors of VEGF (production) and/or of angiogenesis.

In WO 97/48695, WO 97/48696, WO 97/48397, WO 99/31063, WO 99/31060, WO 99/31087, and WO 99/31064 pyridyl alkane, pyridyl alkene and pyridyl alkine acid amides are described, which are referred to as being useful as cytostatics and immunosuppressives, especially for the inhibition of tumor cell growth, preferably for the curative treatment of solid tumors. However no indication is given on the possibility to use these compounds for the inhibition or reduction of VEGF (production) and/or of angiogenesis and/or for the treatment of diseases associated with deregulated angiogenesis.

### Summary of the invention

Surprisingly it has been found that pyridyl amides of Formula I below have advantageous pharmacological properties and inhibit the VEGF production and/or significantly reduce the vessel density.

The compounds of Formula I permit an unexpected new therapeutic approach, especially for diseases in the treatment of which, and also for the prevention of which, an inhibition or reduction of angiogenesis and/or inhibition or reduction of VEGF (production) shows beneficial effects.

The present invention concerns a compound of formula (I) or a pharmaceutical acceptable salt thereof for the prevention or treatment of a disease or medical condition in a mammal, which disease or medical condition responds to inhibition or reduction of angiogenesis and is selected from: rheumatoid arthritis; inflammatory disorder; macular degeneration, especially age-related macular degeneration; retinopathy, especially proliferative retinopathy and diabetic retinopathy; and hyperplasia, especially benign prostatic hyperplasia and venous neointimal hyperplasia, by inhibition or reduction of angiogenesis in a mammal: 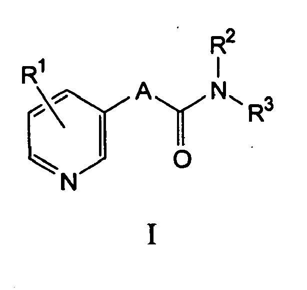 wherein:
A is selected from the group consisting of the group members C₁₋₁₀-alkylene, C₂₋₁₀-alkenylene, and C₂₋₁₀-alkinylene, which group members may be optionally substituted by one, two or three groups independently selected from C₁₋₃-alkyl, fluoro, chloro, and bromo;
R¹ is selected from hydrogen, C₁₋₆-alkyl, fluoro, chloro, bromo, and perfluoro-C₁₋₃-alkyl;
R² is selected from hydrogen, C₁₋₆-alkyl, and C₂₋₆-alkenyl;
R³ is selected from the group consisting of the group members C₁₋₆-alkyl, (C₅₋₈-cycloalkyl)-C₁₋₆-alkyl, (C₅₋₈-heterocyclyl)-C₁₋₆-alkyl, C₁₋₆-alkyl-(C₅₋₈-heterocyclyl)-C₁₋₆-alkyl, and C₁₋₅-alkylcarbonyl-(C₅₋₈-heterocyclyl)-C₁₋₆-alkyl, which group members may be optionally substituted by one, two or three groups independently selected from C₁₋₆-alkyl, fluoro, chloro, bromo, oxo, perfluoro-C₁₋₃-alkyl, aryl, arylcarbonyl, heteroaryl, heteroarylcarbonyl, C₅₋₈-cycloalkyl and C₅₋₈-heterocyclyl.

The present invention furthermore concerns a compound of formula (I) or a pharmaceutical acceptable salt thereof, as defined above, for the prevention or treatment of a disease or medical condition in a mammal, which disease or medical condition responds to VEGF production and is selected from rheumatoid arthritis; inflammatory disorder; macular degeneration, especially age-related macular degeneration; retinopathy, especially proliferative retinopathy and diabetic retinopathy; and hyperplasia, especially benign prostatic hyperplasia and venous neointimal hyperplasia.

Moreover, the present invention concerns the use of a compound of formula (I) or a pharmaceutical salt thereof, as defined above, for the manufacture of a pharmaceutical composition for the prevention or treatment of diseases or medical conditions in a mammal as defined above.

### Brief description of the drawings

Figure 1 shows the influence of a compound according to the invention on the inhibition of VEGF (production) in HepG2 cells.
Figure 2 shows the effect of a compound according to the invention given at lower concentrations on angiogenesis in a murine RENCA model compared to a control and a known antiangiogenic compound.
Figure 3 shows the effect of a compound according to the invention given at increased concentrations on angiogenesis in a murine RENCA model compared to a control and a known antiangiogenic compound.

### Detailed description of the invention

The general terms used herein, within the context of the disclosure, preferably have the following meanings, unless otherwise indicated:

Where compounds of Formula I are mentioned this is taken to mean also the tautomers of the compounds of Formula I.

Where the plural form is used for compounds, salts, this is taken to mean also a single compound, salt

Asymmetric carbon atoms of Formula I that are optionally present may exist in the (R), (S) or (R,S) configuration, preferably in the (R) or (S) configuration. Substituents at a double bond or a ring may be present in cis (i.e. Z) or trans (i.e. E) form, whereas - generally - the trans form is preferred. The compounds may thus be present as mixtures of isomers or as pure isomers, preferably as enantiomer-pure diastereomers.

The activities described for the compounds according to the invention can be observed as long as in Formula I the following requirements are fulfilled: (i) the pyridine ring is substituted in its 3-position (but neither in its 2-position nor in its 4-position), (ii) A does not represent a bond but rather works as a spacer between the pyridine ring and the amide, and (iii) to the spacer A there is attached an amide group (but not an ester group, a thioester group, etc.).

C₁₋₁₀-alkylene, C₂₋₁₀-alkenylene, and C₂₋₁₀-alkinylene as A may be branched or unbranched, and are preferably C₂₋₆-alkylene, C₂₋₆-alkenylene, and C₂₋₄-alkinylene. Examples thereof are ethylene (-CH₂CH₂-), n-propylene (-CH₂CH₂CH₂-), i-propylene (-CH(CH₃)CH₂-), n-butylene (-CH₂CH₂CH₂CH₂-), i-butylene (-CH(CH₃)CH₂CH₂-), n-pentylene (-CH₂CH₂CH₂CH₂CH₂-), n-hexylene (-CH₂CH₂CH₂CH₂CH₂CH₂-), ethenylene (-CH=CH-), 1-propenylene (-CH=CHCH₂-), 1-butenylene (-CH=CHCH₂-), 2-butenylene (-CH₂CH=CHCH₂-), 1-pentenylene (-CH=CHCH₂CH₂CH₂-), 2-pentenylene (-CH₂CH=CHCH₂CH₂-), 1-hexenylene (-CH=CHCH₂CH₂CH₂CH₂-), 2-hexenylene (-CH₂CH=CHCH₂CH₂CH₂-), 3-hexenylene (-CH₂CH₂CH=CHCH₂CH₂-), and ethinylene (-C≡C-), preferred are ethylene, n-propylene, n-butylene, ethenylene and ethinylene, most preferred are ethylene and ethenylene.

When A represents an alkenylene or alkinylene more than one unsaturated bond may be present. In case that more than one unsaturated bond is present in alkenylene it is preferred that these unsaturated bonds are conjugated. Examples thereof are 1,3-butadienylene (-CH=CHCH=CH-), 1,3-pentadienylene (-CH=CHCH=CHCH₂-), 1,3-hexadienylene (-CH=CHCH=CHCH₂CH₂-), and 2,4-hexadienylene (-CH₂CH=CHCH=CHCH₂-). When one or more double bonds are present in A as in alkenylene, e.g. in C₂₋₆-alkenylene, the configuration thereof along the chain connecting the pyridyl and the amide is preferably the E (= trans) form. That is 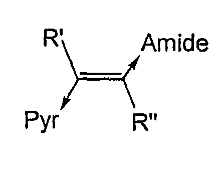 is preferred over 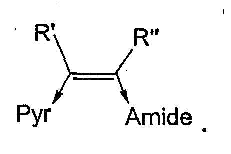 C₁₋₆-alkyl for R¹ and R² is preferably C₁₋₄-alkyl, examples are methyl, ethyl, n-propyl, i-propyl, n-butyl, sec-butyl, and tert-butyl, whereof methyl, ethyl and n-propyl are preferred.

C₂₋₆-alkenyl is preferably C₁₋₄-alkenyl, e.g. ethenyl (which also is called vinyl), 1-propenyl, 2-propenyl, i-propenyl, 1-buten-1-yl, 2-buten-1-yl, 3-buten-1-yl, 1-buten-2-yl, and 2-buten-2-yl, whereof ethenyl, 2-propenyl, and 3-buten-1-yl are preferred, and ethenyl is especially preferred.

The expression perfluoro-C₁₋₃-alkyl relates to C₁₋₃-alkyl wherein all hydrogens are replaced by fluoro atoms, e.g. trifluoromethyl, pentafluoroethyl, and heptafluoropropyl, whereof trifluoromethyl is preferred.

C₁₋₆-alkyl in the definition of R³ may be exemplified by methyl, ethyl, n-propyl, i-propyl, n-butyl, sec-butyl, tert-butyl, n-pentyl, and n-hexyl, whereof ethyl, n-propyl, n-butyl, n-pentyl, and n-hexyl are preferred, wherein n-butyl is especially preferred.

C₅₋₈-cycloalkyl represents a saturated or partially saturated C₅₋₈-cycloalkyl radical. The C₅₋₈-cycloalkyl has 5 to 8, preferably 5 to 7 ring atoms, most preferably 6 ring atoms. Examples of the corresponding C₅₋₈-cycloalkyl rings are cyclopentane, cyclopentene, cyclopentadiene, cyclohexane, cyclohexene, cyclohexadiene, cycloheptane, and cyclooctane, whereof cyclopentane, cyclohexane, cycloheptane, and cyclooctane are preferred, and cyclohexane is most preferred. '

C₅₋₈-heterocyclyl represents a saturated or partially saturated C₅₋₈-heterocyclic radical, whereby one or more, preferably one to four, especially one or two carbon atoms, most preferably one carbon atom of a corresponding C₅₋₈-cycloalkyl radical are/is replaced by the respective number of hetero atoms selected from the group consisting of nitrogen, oxygen and sulfur. The C₅₋₈-heterocyclyl has 5 to 8, preferably 5 to 7 ring atoms, most preferably 6 ring atoms. Examples of the corresponding C₅₋₈-heterocyclic rings are pyrrolidine, 2-pyrroline, 3-pyrroline, imidazolidine, 2-imidazoline, 4-imidazoline, pyrazolidine, 2-pyrazoline, 3-pyrazoline, tetrahydrofurane, tetrahydrothiophene, dihydrofurane, dihydrothiophene, oxazolidine, thiazolidine, piperidine, piperazine, tetrahydropyrane, thiane, morpholine, thiazinane, oxazinane, oxadiazinane, dihydropyridine, oxepane, thiepane, azepane, oxazepane, thiazepane, azocane, and oxazocane, whereof pyrrolidine, piperidine, piperazine, and morpholine are preferred; whereas piperidine and piperazine are especially preferred.

C₁₋₅-alkylcarbonyl refers to a C₁₋₅-alkyl attached to a carbonyl (-(C=O)-) group. Accordingly, it may be exemplified by acetyl, propionyl, butanoyl (= butyryl), i-butanoyl (= 2-methylpropionyl), pentanoyl, and hexanoyl, whereof acetyl, propionyl, and butanoyl are preferred.

The expressions (C₅₋₈-cycloalkyl)-C₁₋₆-alkyl, (C₅₋₈-heterocyclyl)-C₁₋₆-alkyl, C₁₋₆-alkyl-(C₅₋₈-heterocyclyl)-C₁₋₆-alkyl, and C₁₋₅-alkylcarbonyl-(C₅₋₈-heterocyclyl)-C₁₋₆-alkyl used herein accordingly relate to combinations of the respective groups, like (i) (C₅₋₈-cycloalkyl)-C₁₋₆-alkyl means a substitution of a hydrogen in C₁₋₆-alkyl by a C₅₋₈-cycloalkyl, and (ii) C₁₋₆-alkyl-(C₅₋₈-heterocyclyl)-C₁₋₆-alkyl means a substitution of a hydrogen in C₅₋₈-heterocyclyl, being attached to C₁₋₆-alkyl, by a C₁₋₆-alkyl, etc. Thus examples of (i) are 2-cyclohexyl-butyl, 4-cyclopentyl-hexyl, cyclooctyl-methyl, and the like, and of (ii) are 4-(3-methyl-piperidin-4-yl)-butyl, 2-(4-propyl-piperazin-1-yl)-pentyl, 3-(4-butyl-piperazin-1-yl)-hexyl, and the like.

Aryl is preferably an aromatic radical with 6 to 14 carbon atoms, especially phenyl, naphthyl, fluorenyl, or phenanthryl, whereby the said radicals are unsubstituted or substituted by one or more substituents independently of one another, preferably up to three, primarily one or two substituents, especially those selected from unsubstituted, mono- or di-substituted amino, halogen, unsubstituted or substituted alkyl, free, etherified or esterified hydroxy, nitro, cyano, free or esterified carboxy, alkanoyl, unsubstituted, N-mono- or N,N-di-substituted carbamoyl, amidino, guanidino, mercapto, phenylthio, phenylsulfinyl, phenylsulfonyl, ethenyl, phenyl, methylthio, acetyl, methylmercapto (CH₃S-), trifluoromethylmercapto (CF₃S-), trifluoromethylsulfonyl, and methylenedioxy bound to adjacent carbon atoms of the ring; aryl is for example phenyl which is unsubstituted or is substituted by one or two substituents, independently of one another, selected from the group consisting of amino, acetylamino, fluorine, chlorine, bromine, methyl, ethyl, propyl, or t-butyl, trifluoromethyl, hydroxy, methoxy, ethoxy, benzyloxy, and cyano, or (as an alternative or in addition to the above group of substituents) n-decyloxy, carbamoyl, N-methyl- or N-tert-butylcarbamoyl, acetyl, phenyloxy, trifluoromethoxy or 1,1,2,2-tetrafluoroethoxy, ethoxycarbonyl, methyl mercapto, trifluoromethylmercapto, hydroxymethyl, 1-hydroxyethyl, methylsulfonyl, trifluoromethylsulfonyl, phenylsulfonyl, 2-methyl-pyrimidin-4-yl, oxazol-5-yl, 2-methyl-1,3-dioxolan-2-yl, pyrazol-3-yl, methyl-pyrazol-3-yl and methylenedioxy bound to two adjacent carbon atoms; especially preferred are one or two substituents selected independently of one another from methyl, chlorine or bromine, and trifluoromethyl. Aryl in the form of phenyl which is substituted by methylene dioxy is preferably 3,4-methylene dioxyphenyl. Aryl is most preferably phenyl which is preferably unsubstituted or substituted by one or more substituents selected independently of one another from the group consisting of methyl, ethyl, n-propyl, i-propyl, t-butyl, fluorine, chlorine, bromine, methoxy, trifluoromethyl; phenyl is most preferably unsubstituted or substituted by one or two substituents selected independently of one another from the group consisting of methyl, ethyl, isopropyl or t-butyl, bromine, chlorine, fluorine, and trifluoromethyl.

Arylcarbonyl is preferably aryl as defined hereinbefore and hereinafter which is attached to carbonyl. Examples of arylcarbonyl are benzoyl, naphthoyl, fluorenoyl, or phenanthroyl, which are unsubstituted or substituted as explained above for aryl. Accordingly arylcarbonyl is most preferably benzoyl which is preferably unsubstituted or substituted by one or more substituents selected independently of one another from the group consisting of methyl, ethyl, n-propyl, i-propyl, t-butyl, fluorine, chlorine, bromine, methoxy, trifluoromethyl; benzoyl is most preferably unsubstituted or substituted by one or two substituents selected independently of one another from the group consisting of methyl, ethyl, isopropyl or t-butyl, bromine, chlorine, fluorine, and trifluoromethyl. The most important example of arylcarbonyl is benzoyl.

Heteroaryl signifies an unsaturated heterocyclic radical and is mono-, bi- or tricyclic, preferably monocyclic, whereby, one or more, preferably one to four, especially one or two, most preferably one carbon atom(s) of a corresponding aryl radical are replaced by a hetero atom selected from the group consisting of nitrogen, oxygen and sulfur, whereby the binding ring has preferably 4 to 12, especially 5 to 7 ring atoms; whereby heteroaryl is unsubstituted or is substituted by one or more, especially 1 to 3, substituents selected independently from the group consisting of the above-mentioned substituents of aryl; and it is in particular a heteroaryl radical selected from the group consisting of imidazolyl, thienyl, furyl, pyranyl, thianthrenyl, isobenzofuranyl, benzofuranyl, chromenyl, 2H-pyrrolyl, pyrrolyl, imidazolyl, benzimidazolyl, pyrazolyl, thiazolyl, isothiazolyl, oxazolyl, isoxazolyl, pyridyl, pyrazinyl, pyrimidinyl, pyridazinyl, indolizinyl, isoindolyl, 3H-indolyl, indazolyl, triazolyl, tetrazolyl, purinyl, quinolizinyl, isoquinolyl, quinolyl, phthalazinyl, naphthyridinyl, quinoxalyl, quinazolinyl, cinnolinyl, pteridinyl, carbazolyl, phenanthridinyl, acridinyl, perimidinyl, phenanthrolinyl and furazanyl.

Heteroarylcarbonyl is preferably heteroaryl as defined hereinbefore and hereinafter which is attached to carbonyl. Examples of heteroarylcarbonyl are imidazoloyl, thienoyl, furoyl, pyranoyl, thianthrenoyl, isobenzofuranoyl, benzofuranoyl, chromenoyl, 2H-pyrroloyl, pyrroloyl, imidazoloyl, benzimidazoloyl, pyrazoloyl, thiazoloyl, isothiazoloyl, oxazoloyl, isoxazoloyl, pyridoyl, pyrazinoyl, pyrimidinoyl, pyridazinoyl, indolizinoyl, isoindoloyl, 3H-indoloyl, indazoloyl, triazoloyl, tetrazoloyl, purinoyl, quinolizinoyl, isoquinoloyl, quinoloyl, phthalazinoyl, naphthyridinoyl, quinoxaloyl, quinazolinoyl, cinnolinoyl, pteridinoyl, carbazoloyl, phenanthridinoyl, acridinoyl, perimidinoyl, phenanthrolinyl and furazanyl.

When referring to independent substitution by one, two or three groups as e.g. mentioned for A and R³ this means that in the group member to be substituted one, two or three hydrogen atoms are replaced by one, two or three of the substitutes. Accordingly, a C₁₋₆-alkyl group which is independently substituted by fluoro and/or bromo may for example be difluoromethyl, bromo-fluoromethyl, 5-fluoro-3-bromo-1-hexyl. Other combinations in the respective definitions will be clear to a skilled person and only a few exemplifying compounds will be listed, which however are not meant to limit the scope of the invention.

Accordingly, exemplifying compounds for A wherein the group members are substituted are for example 1,1-difluoroethylene, 1,2,3-trifluoropropylene, 1,1-difluorobutylene, 2,3-dibromopentylene, (E)-1,2-difluoroethenylene, (E)-1,2-difluoro-1-butenylene, and 2-fluor-1-propinylene.

Further, exemplifying compounds for R³ wherein the group members are substituted are for example difluoro-C₁₋₆-alkyl, di(perfluoro-C₁₋₃-alkyl)-C₁₋₆-alkyl, (dioxo-C₅₋₈-cycloalkyl)-C₁₋₆-alkyl, [aryl-(dioxo-C₅₋₈-cycloalkyl)]-C₁₋₆-alkyl, (arylcarbonyl-C₅₋₈-cycloalkyl)-C₁₋₆-Alkyl, (arylcarbonyl-C₅₋₈-heterocyclyl)-C₁₋₆-alkyl, [arylcarbonyl-(dioxo-C₅₋₈-heterocyclyl)]-C₁₋₆-alkyl, C₁₋₆-alkyl-(dioxo-C₅₋₈-heterocyclyl)-C₁₋₆-alkyl, (trifluoro-C₁₋₆-alkyl)-(C₅₋₈-heterocyclyl)-C₁₋₆-alkyl, [(diaryl-C₁₋₅-alkyl)carbonyl]-(C₅₋₈-heterocyclyl)-C₁₋₆-alkyl, and the like.

Owing to their efficacy as inhibitors of the VEGF production the compounds according to the present invention inhibit and/or reduce in particular the growth of vessels and are therefore effective against a number of diseases associated with deregulated angiogenesis, such as rheumatoid arthritis; inflammatory disorder; macular degeneration; retinopathy, especially proliferative retinopathy and diabetic retinopathy; and hyperplasia, especially benign prostatic hyperplasia and venous neointimal hyperplasia. The connection of angiogenesis with the above-mentioned diseases is known to a skilled person as it is disclosed for example in the following documents: WO 01/58899; N. Ferrara, J. Mol. Med. (1999) 77: 527-543; D. Walsh et al., Arthritis Res. 2001, 3: 147-153; M. Nagashima, et al., The Journal of Rheumatology 2000, 27: 10, pp. 2339-2342; M. Bhushan, et al., British Journal of Dermatology 1999, 141: pp. 1054-1060; K. Kuroda, et al., The Journal of Investigation Dermatology; Vol. 116, No. 5, pp.713-720; L. Aiello, Current Opinion in Ophthalmology 1997, 8, III: pp. 19-31; G. Chodak, et al., Ann. Surg. 1980, Vol. 192, No. 6, pp. 762-771; K. Weingärtner, The Journal of Urology; Vol. 159, 465-470, 1998; K. Walsh, et al., BJU International (1999), 84, pp. 1081-1083; P. Roy-Chaudhury, et al., Kidney International; Vol. 59 (2001), pp. 2325-2334.

Inhibition and/or reduction of the VEGF (production) as used in the present context is to be understood as such the concentration of VEGF in a mammal or cell(s) thereof is reduced as measured by established (standard) methods, exemplified for example in the Experimental Section. Further well-established determination methods can be used according to the knowledge of a person skilled in the art.

A compound of Formula I can be administered alone or in combination with one or more other therapeutic agents, possible combination therapy taking the form of fixed combinations or the administration of a compound of the invention and one or more other therapeutic agents being staggered or given independently of one another, or the combined administration of fixed combinations and one or more other therapeutic agents.

The amount of the compounds of Formula I to be administered will be determined by a person skilled in the art according to the art. They can be administered as such or in the form of pharmaceutical compositions, prophylactically or therapeutically, preferably in an amount effective against the mentioned diseases and/or conditions, to a warm-blooded animal, for example a human, requiring such treatment, the compounds especially being used in form of pharmaceutical compositions. In the case of an individual having a bodyweight of about 70 kg the daily dose administered is from approximately 1 mg to approximately 5 g, preferably approximately 10 mg to approximately 1 g of a compound of Formula I. It might also be advantageous if the compounds of Formula I are administered several times daily, for example twice or thrice daily. Furthermore, doses administered below the maximum tolerated dose (MTD) might be advantageous, for example doses administered at 25 % to 75 %, especially at 40 to 60% of the MTD might be beneficial.

Compounds according to the invention are not only for the (prophylactic and preferably therapeutic) treatment of humans, but also for the treatment of other warm-blooded animals, for example of commercially useful animals, for example rodents, such as mice, rabbits or rats, or guinea-pigs. They may also be used as a reference standard in the test systems described below in the Experimental Section to permit a comparison with other compounds.

With the groups of preferred compounds of Formula I below, definitions of substituents from the general definitions above may reasonably be used, for example, to replace more general definitions with more specific definitions or especially with definitions characterized as being preferred.

In addition to the above, preference is given to the use of a compound of Formula I, wherein A is selected from ethylene, n-propylene, i-propylene, n-butylene, ethenylene, 1-propenylene, 1-butenylene, 2-butenylene, and ethinylene.

Equally preferred to the above is the use of a compound of Formula I, wherein R¹ is selected from hydrogen, methyl, ethyl, n-propyl, fluoro, and trifluoromethyl.

In addition, the use of a compound of Formula I according to any of the above definitions or combinations of definition is preferred, wherein R² is selected from hydrogen, methyl, ethyl, n-propyl, and ethenyl.

Equally preferred is the use of a compound of Formula I, wherein R³ is selected from the group consisting of the group members cyclopentyl-C₁₋₆-alkyl, cyclohexyl-C₁₋₆-alkyl, pyrrolidinyl-C₁₋₆-alkyl, piperidinyl-C₁₋₆-alkyl, C₁₋₆-alkyl-piperidinyl-C₁₋₆-alkyl, C₁₋₅-alkylcarbonyl-piperidinyl-C₁₋₆-alkyl, piperazinyl-C₁₋₆-alkyl, C₁₋₆-alkyl-piperazinyl-C₁₋₆-alkyl, C₁₋₅-alkylcarbonyl-piperazinyl-C₁₋₆-alkyl, and morpholinyl-C₁₋₆-alkyl, which members may be optionally substituted by one, two or three groups independently selected from C₁₋₆-alkyl, fluoro, chloro, bromo, oxo, perfluoro-C₁₋₃-alkyl, aryl, arylcarbonyl, heteroaryl, C₅₋₈-cycloalkyl, and C₅₋₈-heterocyclyl, and the other residues A, R¹, R² are as defined in any of the above definitions.

It is further preferred the use according to the above, wherein R³ is selected from the group consisting of the group members: cyclohexyl-C₁₋₆-alkyl, piperidinyl-C₁₋₆-alkyl, C₁₋₆-alkyl-piperidinyl-C₁₋₆-alkyl, C₁₋₅-alkylcarbonyl-piperidinyl-C₁₋₆-alkyl, piperazinyl-C₁₋₆-alkyl, C₁₋₆-alkyl-piperazinyl-C₁₋₆-alkyl, C₁₋₅-alkylcarbonyl-piperazinyl-C₁₋₆-alkyl, which members may be optionally substituted by one, two or three groups independently selected from butyl, pentyl, hexyl, fluoro, oxo, phenyl, biphenyl, benzyl, pyridyl, pyrrolyl, benzoyl, thiophenyl, furyl, cyclopentyl, cyclohexyl, and piperidinyl.

According to the use defined hereinbefore it is even more preferred when R³ is selected from the group consisting of
(1-acetyl-piperidin-4-yl)-butyl, (1-diphenylacetyl-piperidin-4-yl)-butyl, [1-(3,3-diphenylpropionyl)-piperidin-4-yl]-butyl, (1-benzoyl-piperidin-4-yl)-ethyl, (1-benzoyl-piperidin-4-yl)-propyl, (1-benzoyl-piperidin-4-yl)-butyl, (1-benzoyl-piperidin-4-yl)-pentyl, (1-benzoyl-piperidin-4-yl)-hexyl, (1-benzylpiperidin-4-yl)-butyl, (1-diphenylmethyl-piperidin-4-yl)-methyl, (1-diphenylmethyl-piperidin-4-yl)-ethyl, (1-diphenylmethyl-piperidin-4-yl)-propyl, (1-diphenylmethyl-piperidin-4-yl)-butyl, (1-diphenylmethyl-piperidin-4-yl)-pentyl, (1-diphenylmethyl-piperidin-4-yl)-hexyl, (4-phenyl-piperidin-1-yl)-butyl, (4,4-diphenyl-piperidin-1-yl)-butyl, (1-benzoyl-2,6-dioxo-piperidin-4-yl)-butyl, (2,6-dioxo-3-phenyl-piperidin-1-yl) -butyl, (2,6-dioxo-4-phenyl-piperidin-1-yl)-butyl, (4-phenyl-piperazin-1-yl)-butyl, (4-phenyl-piperazin-1-yl)-pentyl, (4-phenyl-piperazin-1-yl)-hexyl, (4-diphenylacetyl-piperazin-1-yl)-butyl, (4-benzoylpiperazin-1-yl)-butyl, and (4-benzyl-2,6-dioxo-piperazin-1-yl)-butyl.

Even more preferred is the above use wherein the compound of Formula I is selected from the group consisting of:
N-[4-(1-acetyl-piperidin-4-yl)-butyl]-3-(pyridin-3-yl)-propionamide,
N-[4-(1-acetyl-piperidin-4-yl)-butyl]-3-(pyridin-3-yl)-acrylamide,
N-[4-(1-diphenylacetyl-piperidin-4-yl)-butyl]-3-(pyridin-3-yl)-acrylamide,
N-[4-(1-diphenylacetyl-piperidin-4-yl)-butyl]-3-(pyridin-3-yl)-propionamide,
N-{4-[1-(3,3-diphenylpropionyl)-piperidin-4-yl]-butyl}-3-(pyridin-3-yl)-acrylamide,
N-[3-(1-benzoyl-piperidin-4-yl)-propyl]-3-(pyridin-3-yl)-propionamide,
N-[4-(1-benzoyl-piperidin-4-yl)-butyl]-3-(pyridin-3-yl)-propionamide,
N-[6-(1-benzoyl-piperidin-4-yl)-hexyl]-3-(pyridin-3-yl)-propionamide,
N-[2-(1-benzoyl-piperidin-4-yl)-ethyl]-3-(pyridin-3-yl)-acrylamide,
N-[4-(1-benzoyl-piperidin-4-yl)-butyl]-3-(pyridin-3-yl)-acrylamide,
N-[6-(1-benzoyl-piperidin-4-yl)-hexyl]-3-(pyridin-3-yl)-acrylamide,
N-[4-(1-benzoyl-piperidin-4-yl)-butyl]-5-(pyridin-3-yl)-_{2,4}-pentadienoic acid amide,
N-[4-(4-benzoyl-piperidin-1-yl)-butyl]-3-(pyridin-3-yl)-acrylamide,
N-[4-(4-benzoyl-piperidin-1-yl)-butyl]-3-(pyridin-3-yl)-propionamide,
N-[4-(1-benzylpiperidin-4-yl)-butyl]-3-(pyridin-3-yl)-propionamide,
N-[4-(1-diphenylmethyl-piperidin-4-yl)-butyl]-3-(2-fluoropyridin-3-yl)-propionamide, '
N-[4-(1-diphenylmethyl-piperidin-4-yl)-butyl]-3-(5-fluoropyridin-3-yl)-propionamide,
N-[4-(1-diphenylmethyl-piperidin-4-yl)-butyl]-2-fluoro-3-(pyridin-3-yl)-propionamide,
N-[4-(1-diphenylmethyl-piperidin-4-yl)-butyl]-2,2-difluoro-3-(pyridin-3-yl)-propionamide,
N-[5-(1-diphenylmethyl-piperidin-4-yl)-pentyl]-3-(pyridin-3-yl)-propionamide,
N-[6-(1-diphenylmethyl-piperidin-4-yl)-hexyl]-3-(pyridin-3-yl)-propionamide,
N-[2-(1-diphenylmethyl-piperidin-4-yl)-ethyl]-5-(pyridin-3-yl)-pentanoic acid amide,
N-[4-(1-diphenylmethyl-piperidin-4-yl)-butyl]-3-(pyridin-3-yl)-propionamide,
N-[4-(1-diphenylmethyl-piperidin-4-yl)-butyl]-5-(pyridin-3-yl)-pentanoic acid amide,
N-[2-(1-diphenylmethylpiperidin-4-yl)-ethyl]-5-(pyridin-3-yl)-2,4-pentadienoic acid amide,
N-[4-(1-diphenylmethylpiperidin-4-yl)-butyl]-5-(pyridin-3-yl)-2,4-pentadienoic acid amide,
N-[5-(1-diphenylmethylpiperidin-4-yl)-pentyl]-5-(pyridin-3-yl)-2,4-pentadienoic acid amide,
N-[6-(1-diphenylmethylpiperidin-4-yl)-hexylJ-5-(pyridin-3-yl)-2,4-pentadienoic acid amide,
N-[4-(4-phenyl-piperidin-1-yl)-butyl]-3-pyridin-3-yl-acrylamide,
N-[4-(4,4-diphenyl-piperidin-1-yl)-butyl]-3-pyridin-3-yl-acrylamide,
N-[4-(1-benzoyl-2,6-dioxo-piperidin-4-yl)-butyl]-3-(pyridin-3-yl)-acrylamide,
N-[4-(2,6-dioxo-3-phenyl-piperidin-1-yl)-butyl]-3-pyridin-3-yl-acrylamide,
N-[4-(2,6-dioxo-4-phenyl-piperidin-1-yl)-butyl]-3-pyridin-3-yl-acrylamide,
N-[4-(4-benzoyl-piperazin-1-yl)-butyl]-3-(pyridin-3-yl)-acrylamide,
N-[4-(4-benzoyl-piperazin-1-yl)-butyl]-3-(pyridin-3-yl)-propionamide,
N-[4-(4-diphenylacetyl-piperazin-1-yl)-butyl]-3-pyridin-3-yl-acrylamide,
N-[4-(4-diphenylmethyl-piperazin-1-yl)-butyl]-3-pyridin-3-yl-propionamide,
N-[5-(4-diphenylmethyl-piperazin-1-yl)-pentyl]-3-pyridin-3-yl-acrylamide,
N-[6-(4-diphenylmethyl-piperazin-1-yl)-hexyl]-3-pyridin-3-yl-acrylamide,
N-[4-(4-diphenylmethyl-piperazin-1-yl)-butyl]-2-(pyridin-3-yl)-propionamide,
N-[4-(4-diphenylmethyl-piperazin-1-yl)-butyl]-5-(pyridin-3-yl)-penta-2,4-dienoic acid amide, and
N-[4-(4-benzyl-2,6-dioxo-piperazin-1-yl)-butyl]-3-pyridin-3-yl-acrylamide.

Most preferred is the above use of N-[4-(1-benzoyl-piperidin-4-yl)-butyl]-3-(pyridin-3-yl)-acrylamide.

All these compounds can be prepared according to standard synthetic routes as they are disclosed for example in the patent documents: WO 97/48695, WO 97/48696, WO 97/48397, WO 99/31063, WO 99/31060, WO 99/31087, and WO 99/31064 as well as in the references cited in these publications. Again it is stressed that the use according to the present invention is different from the use described in these publications.

In the following, examples are presented to show the high efficacy of the compounds of the invention in the reduction and/or inhibition of VEGF (production) as well as in the reduction and/or inhibition of angiogenesis. These examples are meant to further illustrate the invention

### Therapeutic Administration Forms

The production of pharmaceutical compositions with an amount of one or more compounds according to the invention occurs in the customary manner by means of common pharmaceutical technology methods. For this, the active ingredients as such or in the form of their salts are processed together with suitable, pharmaceutically acceptable adjuvants and carriers to medicinal forms suitable for the various indications and types of application. Thereby, the pharmaceutical compositions can be produced in such a manner that the respective desired release rate is obtained, for example a quick flooding and/or a sustained or depot effect.

Preparations for parenteral use, to which injections and infusions belong, are among the most important systemically employed pharmaceutical compositions.

Injections are prepared either in the form of vials or also as so-called ready-to-use injection preparations, for example as ready-to-use syringes or single use syringes in addition to perforation bottles for multiple withdrawals. Administration of the injection preparations can occur in the form of subcutaneous (s.c.), intramuscular (i.m.), intravenous (i.v.) or intracutaneous (i.e.) application. The respective suitable injection forms can especially be produced as solutions, crystal suspensions, nanoparticular or colloid-disperse systems, such as for example, hydrosols.

The injectable formulations can also be produced as concentrates which can be adjusted with aqueous isotonic dilution agents to the desired active ingredient dosage. Furthermore, they can also be produced as powders, such as for example lyophilisates, which are then preferably dissolved or dispersed immediately before application with suitable diluents. The infusions can also be formulated in the form of isotonic solutions, fat emulsions, liposome formulations, microemulsions and liquids based on mixed micelles, for example, based on phospholipids. As with injection preparations, infusion formulations can also be prepared in the form of concentrates to dilute. The injectable formulations can also be applied in the form of continuous infusions as in stationary as well as in outpatient therapy, for example in the form of mini-pumps.

Albumin, plasma expanders, surface active compounds, organic solvents, pH influencing compounds, complex forming compounds or polymeric compounds can be added to the parenteral medicinal forms, especially as substances for influencing the adsorption of the active ingredients to protein or polymers or also with the aim of decreasing the adsorption of the active ingredient to materials such as injection instruments or packaging materials, for example plastic or glass.

The active ingredients can be bound to nanoparticles in the preparations for parenteral use, for example on finely dispersed particles based on poly(meth)acrylates, polyacetates, polyglycolates, polyamino acids or polyether urethanes. The parenteral formulations can also be constructively modified as depot preparations, for example on the multiple unit principle, where the active ingredients are incorporated in a most finely distributed and/or dispersed, suspended form or as crystal suspensions, or on the single unit principle, where the active ingredient is enclosed in a medicinal form, for example, a tablet or a seed which is subsequently implanted. Often, these implantations or depot pharmaceutical compositions in single unit and multiple unit medicinal forms consist of so-called biodegradable polymers, such as for example, polyether urethanes of lactic and glycolic acid, polyether urethanes, polyamino acids, poly(meth)acrylates or polysaccharides.

Sterilized water, pH value influencing substances, such as for example organic and inorganic acids or bases as well as their salts, buffer substances for setting the pH value, agents for isotonicity, such as for example sodium chloride, monosodium carbonate, glucose and fructose, tensides and/or surface active substances and emulsifiers, such as for example, partial fatty acid esters of polyoxyethylene sorbitan (Tween®) or for example fatty acid esters of polyoxethylene (Cremophor®), fatty oils such as for example peanut oil, soybean oil and castor oil, synthetic fatty acid esters, such as for example ethyl oleate, isopropyl myristate and neutral oil (Miglyol®) as well as polymer adjuvants such as for example gelatin, dextran, polyvinylpyrrolidone, organic solvent additives which increase solubility, such as for example propylene glycol, ethanol, N,N-dimethylacetamide, propylene glycol or complex forming compounds such as for example citrates and urea, preservatives, such as for example hydroxypropyl benzoate and hydroxymethyl benzoate, benzyl alcohol, anti-oxidants, such as for example sodium sulfite and stabilizers, such as for example EDTA, are suitable as adjuvants and carriers in the production of preparations for parenteral use.

In suspensions, addition of thickening agents to prevent the settling of the active ingredients from tensides and peptizers, to secure the ability of the sediment to be shaken, or complex formers, such as EDTA, ensues. This can also be achieved with the various polymeric agent complexes, for example with polyethylene glycols, polystyrol, carboxymethylcellulose, Pluronics® or polyethylene glycol sorbitan fatty acid esters. The active ingredient can also be incorporated in liquid formulations in the form of inclusion compounds, for example with cyclodextrins. As further adjuvants, dispersion agents are also suitable. For production of lyophilisates, builders are also used, such as for example mannite, dextran, saccharose, human albumin, lactose, PVP or gelatin varieties.
As long as the active ingredients are not incorporated in the liquid medicinal formulations in the form of a base, they are used in the form of their acid addition salts, hydrates or solvates in the preparations for parenteral use.

A further systemic application form of importance is peroral administration as tablets, hard or soft gelatin capsules, coated tablets, powders, pellets, microcapsules, oblong compressives, granules, chewable tablets, lozenges, gums or sachets. These solid peroral administration forms can also be prepared as sustained action and/or depot systems. Among these are pharmaceutical compositions with an amount of one or more micronized active ingredients, diffusions and erosion forms based on matrices, for example by using fats, wax-like and/or polymeric compounds, or so-called reservoir systems. As a retarding agent and/or agent for controlled release, film or matrix forming substances, such as for example ethylcellulose, hydroxypropylmethylcellulose, poly(meth)acrylate derivatives (for example Eudragit®), hydroxypropylmethylcellulose phthalate are suitable in organic solutions as well as in the form of aqueous dispersions. In this connection, so-called bio-adhesive preparations are also to be named in which the increased retention time in the body is achieved by intensive contact with the mucus membranes of the body. An example of a bio-adhesive polymer is the group of Carbomers®.

For sublingual application, compressives, such as for example non-disintegrating tablets in oblong form of a suitable size with a slow release of active ingredient, are especially suitable. For purposes of a targeted release of active ingredients in the various sections of the gastrointestinal tract, mixtures of pellets which release at the various places are employable, for example mixtures of gastric fluid soluble and small intestine soluble and/or gastric fluid resistant and large intestine soluble pellets. The same goal of releasing at various sections of the gastrointestinal tract can also be conceived by suitably produced laminated tablets with a core, whereby the coating of the agent is quickly released in gastric fluid and the core of the agent is slowly released in the small intestine milieu. The goal of controlled release at various sections of the gastrointestinal tract can also be attained by multilayer tablets. The pellet mixtures with differentially released agent can be filled into hard gelatin capsules.

Anti-stick and lubricant and separating agents, dispersion agents such as flame dispersed silicone dioxide, disintegrants, such as various starch types, PVC, cellulose esters as granulating or retarding agents, such as for example wax-like and/or polymeric compounds on the basis of Eudragit®, cellulose or Cremophor® are used as a further adjuvants for the production of compressives, such as for example tablets or hard and soft gelatin capsules as well as coated tablets and granulates.

Anti-oxidants, sweetening agents, such as for example saccharose, xylite or mannite, masking flavors, aromatics, preservatives, colorants, buffer substances, direct tableting agents, such as for example microcrystalline cellulose, starch and starch hydrolysates (for example Celutab®), lactose, polyethylene glycols, polyvinylpyrrolidone and dicalcium phosphate, lubricants, fillers, such as lactose or starch, binding agents in the form of lactose, starch varieties, such as for example wheat or corn and/or rice starch, cellulose derivatives, for example methylcellulose, hydroxypropylcellulose or silica, talcum powder, stearates, such as for example magnesium stearate, aluminum stearate, calcium stearate, talc, siliconized talc, stearic acid, acetyl alcohol and hydrated fats are used.

In this connection, oral therapeutic systems constructed especially on osmotic principles, such as for example GIT (gastrointestinal therapeutic system) or OROS (oral osmotic system), are also to be mentioned.

Effervescent tablets or tabs both of which represent immediately drinkable instant medicinal forms which are quickly dissolved or suspended in water are among the perorally administratable compressives. Among the perorally administratable forms are also solutions, for example drops, juices and suspensions, which can be produced according to the above given method, and can still contain preservatives for increasing stability and optionally aromatics for reasons of easier intake, and colorants for better differentiation as well as antioxidants and/or vitamins and sweeteners such as sugar or artificial sweetening agents. This is also true for inspisated juices which are formulated with water before ingestion. Ion exchange resins in combination with one or more active ingredients are also to be mentioned for the production of liquid ingestable forms.

A special release form consists in the preparation of so-called floating medicinal forms, for example based on tablets or pellets which develop gas after contact with body fluids and therefore float on the surface of the gastric fluid. Furthermore, so-called electronically controlled release systems can also be formulated by which active ingredient release can be selectively adjusted to individual needs.

A further group of systemic administration and also optionally topically effective medicinal forms are represented by rectally applicable pharmaceutical compositions. Among these are suppositories and enema formulations. The enema formulations can be prepared based on tablets with aqueous solvents for producing this administration form. Rectal capsules can also be made available based on gelatin or other carriers.

Hardened fat, such as for example Witepsol®, Massa Estarinum®, Novata®, coconut fat, glycerol-gelatin masses, glycerol-soap-gels and polyethylene glycols are suitable as suppository bases.

For long-term application with a systematic active ingredient release up to several weeks, pressed implants are suitable which are preferably formulated on the basis of so-called biodegradable polymers.

As a further important group of systemically active pharmaceutical compositions, transdermal systems are also to be emphasized which distinguish themselves, as with the above-mentioned rectal forms, by circumventing the liver circulation system and/or liver metabolism. These plasters can be especially prepared as transdermal systems which are capable of releasing the active ingredient in a controlled manner over longer or shorter time periods based on different layers and/or mixtures of suitable adjuvants and carriers. Aside from suitable adjuvants and carriers such as solvents and polymeric components, for example based on Eudragit®, membrane infiltration increasing substances and/or permeation promoters, such as for example oleic acid, Azone®, adipinic acid derivatives, ethanol, urea, propylglycol are suitable in the production of transdermal systems of this type for the purpose of improved and/or accelerated penetration.

As topically, locally or regionally administration pharmaceutical compositions, the following are suitable as special formulations: vaginally or genitally applicable emulsions, creams, foam tablets, depot implants, ovular or transurethral administration installation solutions. For ophthalmological application, highly sterile eye ointments, solutions and/or drops or creams and emulsions are suitable.

In the same manner, corresponding otological drops, ointments or creams can be designated for application to the ear. For both of the above-mentioned applications, the administration of semi-solid formulations, such as for example gels based on Carbopols® or other polymer compounds such as for example polyvinylpyrolidone and cellulose derivatives is also possible.

For customary application to the skin or also to the mucus membrane, normal emulsions, gels, ointments, creams or mixed phase and/or amphiphilic emulsion systems (oil/water-water/oil mixed phase) as well as liposomes and transfersomes can be named. Sodium alginate as a gel builder for production of a suitable foundation or cellulose derivatives, such as for example guar or xanthene gum, inorganic gel builders, such as for example aluminum hydroxides or bentonites (so-called thixotropic gel builder), polyacrylic acid derivatives, such as for example Carbopol®, polyvinylpyrolidone, microcrystalline cellulose or carboxymethylcellulose are suitable as adjuvants and/or carriers. Furthermore, amphiphilic low and high molecular weight compounds as well as phospholipids are suitable. The gels can be present either as hydrogels based on water or as hydrophobic organogels, for example based on mixtures of low and high molecular paraffin hydrocarbons and vaseline.

Anionic, cationic or neutral tensides can be employed as emulsifiers, for example alkalized soaps, methyl soaps, amine soaps, sulfonated compounds, cationic soaps, high fatty alcohols, partial fatty acid esters of sorbitan and polyoxyethylene sorbitan, for example lanette types, wool wax, lanolin, or other synthetic products for the production of oil/water and/or water/oil emulsions.

Hydrophilic organogels can be formulated, for example, on the basis of high molecular polyethylene glycols. These gel-like forms are washable. Vaseline, natural or synthetic waxes, fatty acids, fatty alcohols, fatty acid esters, for example as mono-, di-, or triglycerides, paraffin oil or vegetable oils, hardened castor oil or coconut oil, pig fat, synthetic fats, for example based on acrylic, caprinic, lauric and stearic acid, such as for example Softisan® or triglyceride mixtures such as Miglyol® are employed as lipids in the form of fat and/or oil and/or wax-like components for the production of ointments, creams or emulsions.

Osmotically effective acids and bases, such as for example hydrochloric acid, citric acid, sodium hydroxide solution, potassium hydroxide solution, monosodium carbonate, further buffer systems, such as for example citrate, phosphate, Tris-buffer or triethanolamine are used for adjusting the pH value.

Preservatives, for example such as methyl- or propyl benzoate (parabenes) or sorbic acid can be added for increasing stability.

Pastes, powders or solutions are to be mentioned as further topically applicable forms. Pastes often contain lipophilic and hydrophilic auxiliary agents with very high amounts of fatty matter as a consistency-giving base.

Powders or topically applicable powders can contain for example starch varieties such as wheat or rice starch, flame dispersed silicon dioxide or silica, which also serve as diluents, for increasing flowability as well as lubricity as well as for preventing agglomerates.

The respective suitable medicinal forms can be produced in accordance with the prescription and procedures based on pharmaceutical-physical fundamentals as they are described for example in the following handbooks.

Physical Pharmacy (A.N. Martin, J. Swarbrick, A. Cammarata), 2nd Ed., Philadelphia Pennsylvania, (1970), German version: Physikalische Pharmazie, (1987), 3rd edition, Stuttgart;
R. Voigt, M. Bornschein, Lehrbuch der pharmazeutischen Technologie, Verlag Chemie, Weinheim, (1984), 5th edition;
P.H. List, Arzneimformenlehre, Wissenschaftliche Verlagsgesellschaft mbH, Stuttgart, (1985), 4th edition;
H. Sucker, P. Fuchs, P. Speiser, Pharmazeutische Technologie, Georg Thieme Verlag, Stuttgart - New York, (1991), 2nd edition;
A.T. Florence, D. Attwood, Physicochemical Principles of Pharmacy, The Maximillan Press Ltd., Hong Kong, (1981);
L.A. Trissel, Handbook on Injectable Drugs, American' Society of Hospital Pharmacists, (1994), 8th edition;
Y.W. Chien, Transdermal Controlled Systemic Medications, Marcel Dekker Inc., New York - Basel, (1987);
K.E. Avis, L. Lachmann, H.A. Liebermann, Pharmaceutical Dosage Forms: Parenteral Medications, volume 2, Marcel Dekker Inc., New York - Basel, (1986);
B.W. Müller, Controlled Drug Delivery, Paperback APV, volume 17, Wissenschaftliche Verlagsgesellschaft mbH, Stuttgart, (1987);
H. Asch, D. Essig, P.C. Schmidt, Technologie von Salben, Suspensionen und Emulsionen, Wissenschaftliche Verlagsgesellschaft mbH, Stuttgart, (1984);
H.A. Liebermann, L. Lachman, J.B. Schwartz, Pharmaceutical Dosage forms: Tablets, Volume 1, Marcel Dekker Inc., New York, 2nd Edition (1989);
D. Chulin, M. Deleuil, Y. Pourcelot, Powder Technology and Pharmaceutical Processes, in J.C. Williams, T. Allen, Handbook of Powder Technology, Elsevier Amsterdam - London - New York - Tokyo, (1994);
J.T. Carstensen, Pharmaceutical Principles of Solid Dosage Forms, Technomic Publishing Co., Inc., Lancaster - Basel, (1993).

### Experimental Section

Effect of a compound according to the invention on VEGF production in HepG2 cells

### Materials and Methods

1. Cell Culture
1.1 Reagents
   Trypsin/EDTA: 0.05 % (w/v) trypsin (Difco, Detroit, USA) + 0.016 % (w/v) EDTA (Sigma, Deisenhofen, Germany). N-[4-(1-benzoyl-piperidin-4-yl)-butyl]-3-(pyridin-3-yl)-acrylamide (in the following also referred to as BPPA) was synthesized as described in the literature, by the department of chemistry at Klinge Pharma GmbH, Munich, Germany.
   Stock solutions of test compound: a 10 mM solution was prepared in dimethyl sulfoxide (DMSO) and stored at -18°C; further dilution steps were done in ethanol.
1.2 Cell lines
   The human-derived tumor cell lines HepG2 (liver carcinoma) was obtained from the American Type Culture Collection (ATCC), Rockville, Maryland, USA.
1.2.1 Growth media
   Richter's Improved Minimal Essential Medium, Zinc Option (IMEM-ZO), was purchased from Gibco BRL, Life Technologies (Eggenstein, Germany). The standard medium MEM and the human serum albumin were purchased from Sigma (Deisenhofen, Germany). The medium powder was dissolved in deionized water, the pH titrated to 7.2 with HCl / NaOH and sterilized by filtration. The medium was supplemented with 10 % fetal calf serum (FCS), PAN Systems GmbH, Aidenbach, Germany.
1.2.2 Methods
   Adherent cell line: Hep G2
   Monolayer cells were detached from 75 cm² flasks by removing the growth medium and adding 3 ml trypsin/EDTA solution to each flask. After about 5-10 minutes incubation at 37°C, when the cells were detached from the surface of the dishes, trypsinization was stopped by adding 3 ml Richter's IMEM-ZO medium containing 10 % FCS. The cells were suspended by repeated pipetting. For predilution, an aliquot of 20 µl was added to 10 ml Casyton isotonic solution (No. 043-90037P, Schärfe System, Reutlingen, Germany) using a Sysmex Auto Dilutor Type AD-260 (Toa, Medical Electronics Co. Ltd., Kobe, Japan). The cell number was determined by electronic cell volume measurements by counting with a CASY 1 Cell Counter & Analyzer System, Model TTC (Schärfe System, Reutlingen, Germany) equipped with a 60 µm capillary. Following dilution in growth medium (IMEM-ZO), the cells were finally seeded at a density of 25,000 cells per 1000 µl in 24-well culture dishes (Greiner, Frickenhausen, Germany) for the VEGF assay and incubated at 37°C in a humidified atmosphere of 5 % CO₂ in air.

After six days of incubation, when the cells were nearly confluent, the medium was removed and cultures were replenished with 1 ml fresh serum free MEM medium containing 0.1 % human serum albumin plus the test substance or the solvent ethanol as control. The cells were then incubated at 37°C in a humidified atmosphere of 5 % CO₂ in air. On the next day the medium was removed again and the cultures were incubated with further 0.5 ml serum free MEM containing 0.1 % human serum albumin plus the test substance or the solvent ethanol as control. After 48 and 72 hours the medium was collected, centrifuged at 1000 rpm for 5 minutes to remove cell debris. Supernatants were stored at -20°C until quantification of the VEGF concentration. Furthermore the protein content of the remaining cell layer in each well was determined as reference parameter.

VEGF determination was measured in the supernatants of the cell cultures using a commercially available ELISA kit (R&D, Systems, Wiesbaden/Germany). The method for the determination was performed as described in the test kit brochure.

The results are summarized in the following Table 1, where the VEGF concentration in the supernatant after 48 h and 72 h of incubation was determined. Values of the untreated controls are compared with compound-treated cells where N-[4- (1-benzoyl-piperidin-4-yl) -butyl] -3- (pyridin-3-yl) - acrylamide was present in the medium in concentrations of 2 nanomole/l and 20 nanomole/l.

**Table 1: VEGF concentration in pg/200 µl after 48 h and 72 h of incubation of untreated controls compared to treated cells, where N-[4-(1-benzoyl-piperidin-4-yl)-butyl]-3-(pyridin-3-yl)-acrylamide was present in the medium in concentrations of 2 nanomole/l and 20 nanomole/l.**

| | 48 hours | 72 hours |
|---|---|---|
| Control | 853.89 | 880.92 |
| 2 nM | 208.30 | 0.00 |
| 20 nM | 0.00 | 0.00 |

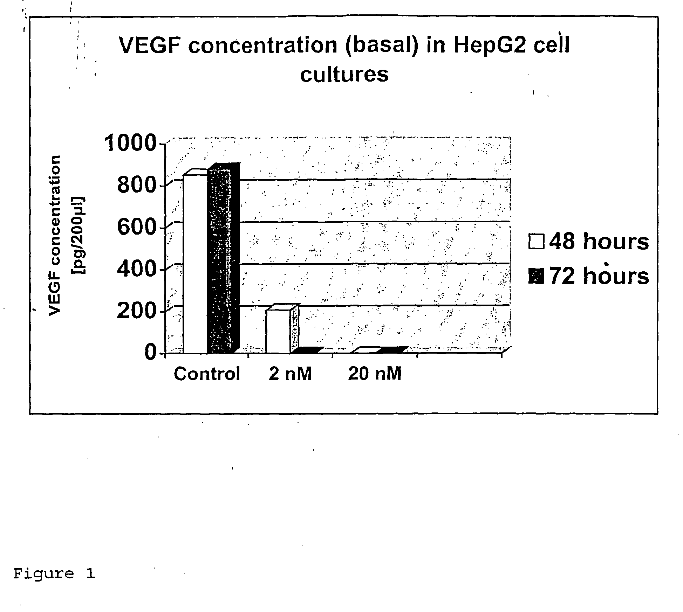

These results are further illustrated in Figure 1 showing that N-[4-(1-benzoyl-piperidin-4-yl)-butyl]-3-(pyridin-3-yl)-acrylamide (BPPA) inhibited the production of VEGF in HepG2 cells very effectively already after 48 hours of incubation.

### Effect of a compound according to the invention on angiogenesis in a murine renal cell carcinoma (RENCA) model

### Materials and Methods

### 1. Cell line and cell culture

Murine RENCA cells were originally obtained from a tumor that arose spontaneously in the kidney of BALB/c mice. Histologically, RENCA consists of granular cell type adenocarcinoma, which is pleomorphic with large nuclei. Monolayers of murine RENCA cells were grown in RPMI 1640 with phenol red supplemented with 10% FCS, 2 mM L-glutamine, 100 units penicillin/ml, and 100 µg of streptomycin/ml. RENCA cells were cultured in a humidified atmosphere of 95% air and 5% carbon dioxide at 37°C. Media were routinely changed every 3 days.

### Morphology: epithelial cells, growing adherently as monolayer

Subculture: split confluent cultures 1:5 to 1:10 every 3-4 days using trypsin/EDTA, cells grow easily, seed out at ca. 1-2 x 10⁶ cells/80 cm² + 10 ml medium
Incubation: at 37°C with 5% CO₂
Doubling Time: doubling time of ca. 48 hours
Storage : frozen with 70% medium, 20% FCS, 10% DMSO

### 2. Test substances

N- [4- (1-Benzoyl-piperidin-4-yl) -butyl] -3- (pyridin-3-yl) - acrylamide was synthesized as described in the literature, by the department of chemistry at Klinge Pharma GmbH, Munich, Germany.
Antiangiogenic compound TNP-470 was obtained from Takeda Chemical Industries, Ltd. (Osaka,Japan).
For the in vivo administration N-[4-(1-benzoyl-piperidin-4-yl)-butyl]-3-(pyridin-3-yl)-acrylamide was solubilized in a 0.9 % saline solution and TNP-470 in a saline solution containing 3 % absolute ethanol.

### 3. Animals

All experiments were carried out using 6-8 weeks old male Balb/c mice (approximate weight, 20 g). Mice are maintained in separated conventional housing at constant temperature and humidity

### 4. Tumor implantation

On each control or drug-treated group the RENCA tumor cells were implanted in the kidney of the mice. The injection of 10⁶ cells in 0.2 ml aliquots into the subcapsular space of the left kidney was performed through a flank incision after the animals were anesthetized with 1.0-1.5 volume percent isoflurane, which is used in combination with an oxygen flow of 2 1/min. The subcapsular renal injection of 10⁶ RENCA cells in a syngeneic Balb/c mouse is followed by the progressive development of a primary tumor mass in the left kidney. One week after application, the primary tumor is macroscopically visible.

### 5. Drug treatment

N-[4-(1-benzoyl-piperidin-4-yl)-butyl]-3-(pyridin-3-yl)-acrylamide was given to the animals p.o. twice a day on days 10, 11, 12, 13, 14, and 15 after tumor implantation. The doses were 3, 5, 6, or 10 mg/kg per each administration. TNP-470 was injected into the animals s.c. on days 2, 4, 6, 8, 10, 12, 14, 16, 18 and 20 after tumor implantation at the maximum tolerated dose, 30mg/kg. Control animals received the vehicle (0.9 % saline solution) p.o. twice a day on days 10, 11, 12, 13, 14, and 15 after tumor implantation. At least seven animals per test group were used.

### 6. Determination of blood vessel density

All animals were sacrificed on day 21 after tumor implantation and the tumor tissue of all animals was surgically removed and quickly frozen in liquid nitrogen. For histological examination of the tumor vasculature, cryosections of the tissues with a thickness of 5-10 µm were taken from all treatment groups and control groups. For the visualization of the blood vessels, immunohistochemical staining for CD 31 (PECAM-1 and MEC 13.3; PharMingen, San Diego, CA) was performed, and vessels were counted microscopically using a defined magnification.

### 7. Results

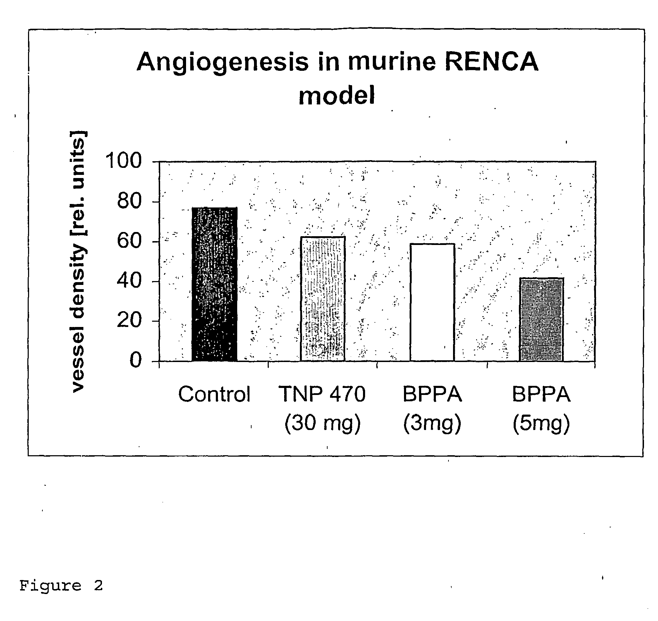

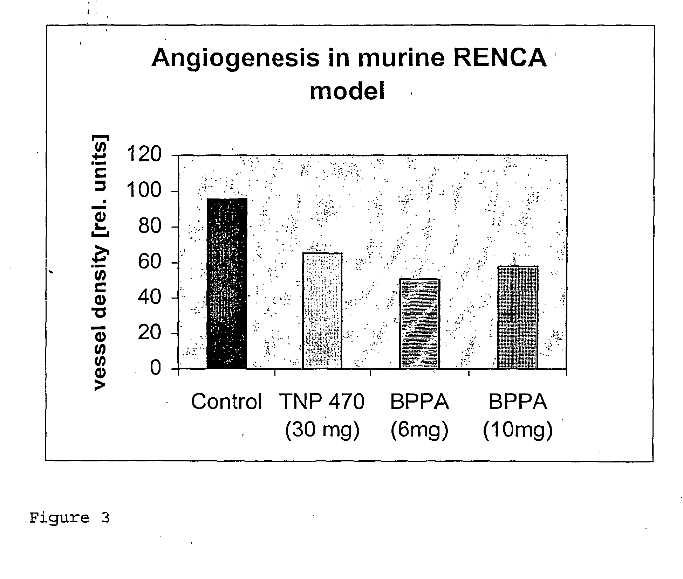

Figure 2 and 3 illustrate the effect on the vessel density in primary tumors of murine renal cell carcinoma (RENCA) of N-[4-(1-benzoyl-piperidin-4-yl)-butyl]-3-(pyridin-3-yl)-acrylamide (BPPA) given orally as outlined above compared to TNP-470 and a control. Primary tumor tissues were processed as explained above under item 6.

TNP-470 was administered at the MTD (maximum tolerated dose), 30 mg/kg, and over a longer time range than BPPA which was given below the MTD (approximately 12 mg/kg). Still TNP-470 was not as effective as BPPA, which in dosages of 3, 5, 6 and 10 mg/kg significantly reduced the vessel density in the RENCA tumor.

## Claims

1. Use of a compound of Formula I or a pharmaceutically acceptable salt thereof for the manufacture of a pharmaceutical composition for the prevention or treatment of a disease or medical condition in a mammal, which disease or medical condition responds to inhibition or reduction of angiogenesis and is selected from rheumatoid arthritis; inflammatory disorder; macular degeneration, especially age-related macular degeneration; retinopathy, especially proliferative retinopathy and diabetic retinopathy; and hyperplasia, especially benign prostatic hyperplasia and venous neointimal hyperplasia: 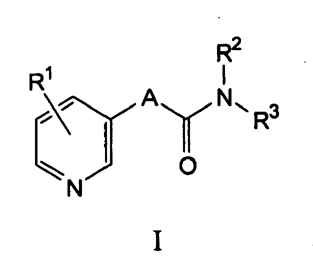 wherein:
A is selected from the group consisting of the group members C₁₋₁₀-alkylene, C₂₋₁₀-alkenylene, and C₂₋₁₀-alkinylene, which group members may be optionally substituted by one, two or three groups independently selected from C₁₋₃-alkyl, fluoro, chloro, and bromo;
R¹ is selected from hydrogen, C₁₋₆-alkyl, fluoro, chloro, bromo, and perfluoro-C₁₋₃-alkyl;
R² is selected from hydrogen, C₁₋₆-alkyl, and C₂₋₆-alkenyl; R³ is selected from the group consisting of the group members C₁₋₆-alkyl, (C₅₋₈-cycloalkyl)-C₁₋₆-alkyl, (C₅₋₈-heterocyclyl)-C₁₋₆-alkyl, C₁₋₆-alkyl-(C₅₋₈-heterocyclyl)-C₁₋₆-alkyl, and C₁₋₅-alkylcarbonyl-(C₅₋₈-heterocyclyl)-C₁₋₆-alkyl, which group members may be optionally substituted by one, two or three groups independently selected from C₁₋₆-alkyl, fluoro, chloro, bromo, oxo, perfluoro-C₁₋₃-alkyl, aryl, arylcarbonyl, heteroaryl, heteroarylcarbonyl, C₅₋₈-cycloalkyl and C₅₋₈-heterocyclyl.

2. Use according to claim 1, wherein A is selected from ethylene, n-propylene, i-propylene, n-butylene, ethenylene, 1-propenylene, 1-butenylene, 2-butenylene, and ethinylene.

3. Use according to claim 1 or 2, wherein R¹ is selected from hydrogen, methyl, ethyl, n-propyl, fluoro, and trifluoromethyl.

4. Use according to any one of claims 1 to 3, wherein R² is selected from hydrogen, methyl, ethyl, n-propyl, and ethenyl.

5. Use according to any one of claims 1 to 4, wherein R³ is selected from the group consisting of the group members cyclopentyl-C₁₋₆-alkyl, cyclohexyl-C₁₋₆-alkyl, pyrrolidinyl-C₁₋₆-alkyl, piperidinyl-C₁₋₆-alkyl, C₁-₆-alkyl-piperidinyl-C₁₋₆-alkyl, C₁₋₅-alkylcarbonyl-piperidinyl-C₁₋₆-alkyl, piperazinyl-C₁₋₆-alkyl, C₁₋₆-alkyl-piperazinyl-C₁₋₆-alkyl, C₁₋₅-alkylcarbonyl-piperazinyl-C₁₋₆-alkyl, and morpholinyl-C₁₋₆-alkyl, which members may be optionally substituted by one, two or three groups independently selected from C₁₋₆-alkyl, fluoro, chloro, bromo, oxo, perfluoro-C₁₋₃-alkyl, aryl, arylcarbonyl, heteroaryl, C₅₋₈-cycloalkyl, and C₅₋₈-heterocyclyl.

6. Use according to any one of claims 1 to 5, wherein R³ is selected from the group consisting of the group members: cyclohexyl-C₁₋₆-alkyl, piperidinyl-C₁₋₆-alkyl, C₁₋₆-alkyl-piperldinyl-C₁₋₆-alkyl, C₁₋₅-alkylcarbonyl-piperidinyl-C₁₋₆-alkyl, piperazinyl-C₁₋₆-alkyl, C₁₋₆-alkyl-piperazinyl-C₁₋₆-alkyl, C₁₋₅-alkylcarbonyl-piperazinyl-C₁₋₆-alkyl, which members may be optionally substituted by one, two or three groups independently selected from butyl, pentyl, hexyl, fluoro, oxo, phenyl, biphenyl, benzyl, pyridyl, pyrrolyl, benzoyl, thiophenyl, furyl, cyclopentyl, cyclohexyl, and piperidinyl.

7. Use according to any one of claims 1 to 6, wherein R³ is selected from the group consisting of
(1-acetyl-piperidin-4-yl)-butyl, (1-diphenylacetyl-piperidin-4-yl)-butyl, [1-(3,3-diphenylpropionyl)-piperidin-4-yl]-butyl, (1-benzoyl-piperidin-4-yl)-ethyl, (1-benzoyl-piperidin-4-yl)-propyl, (1-benzoyl-piperidin-4-yl)-butyl, (1-benzoyl-piperidin-4-yl)-pentyl, (1-benzoyl-piperidin-4-yl)-hexyl, (1-benzylpiperidin-4-yl)-butyl, (1-diphenylmethyl-piperidin-4-yl)-methyl, (1-diphenylmethyl-piperidin-4-yl)-ethyl, (1-diphenylmethyl-piperidin-4-yl)-propyl, (1-diphenylmethyl-piperidin-4-yl)-butyl, (1-diphenylmethyl-piperidin-4-yl)-pentyl, (1-diphenylmethyl-piperidin-4-yl)-hexyl, (4-phenyl-piperidin-1-yl)-butyl, (4,4-diphenyl-piperidin-1-yl)-butyl, (1-benzoyl-2,6-dioxo-piperidin-4-yl)-butyl, (2,6-dioxo-3-phenyl-piperidin-1-yl)-butyl, (2,6-dioxo-4-phenyl-piperidin-1-yl)-butyl, (4-phenyl-piperazin-1-yl)-butyl, (4-phenyl-piperazin-1-yl)-pentyl, (4-phenyl-piperazin-1-yl)-hexyl, (4-diphenylacetyl-piperazin-1-yl)-butyl, (4-benzoylpiperazin-1-yl)-butyl, and (4-benzyl-2,6-dioxo-piperazin-1-yl)-butyl.

8. Use according to any one of claims 1 to 7, wherein the compound of Formula I is selected from the group consisting of
N-[4-(1-acetyl-piperidin-4-yl)-butyl]-3-(pyridin-3-yl)-propionamide,
N-[4-(1-acetyl-piperidin-4-yl)-butyl]-3-(pyridin-3-yl)-acrylamide,
N-[4-(1-diphenylacetyl-piperidin-4-yl)-butyl]-3-(pyridin-3-yl)-acrylamide,
N-[4-(1-diphenylacetyl-piperidin-4-yl)-butyl]-3-(pyridin-3-yl)-propionamide,
N-{4-[1-(3,3-diphenylpropionyl)-piperidin-4-yl]-butyl}-3-(pyridin-3-yl)-acrylamide,
N-[3-(1-benzoyl-piperidin-4-yl)-propyl]-3-(pyridin-3-yl)-propionamide,
N-[4-(1-benzoyl-piperidin-4-yl)-butyl]-3-(pyridin-3-yl)-propionamide,
N-[6-(1-benzoyl-piperidin-4-yl)-hexyl]-3-(pyridin-3-yl)-propionamide,
N-[2-(1-benzoyl-piperidin-4-yl)-ethyl]-3-(pyridin-3-yl)-acrylamide,
N-[4-(1-benzoyl-piperidin-4-yl)-butyl]-3-(pyridin-3-yl)-acrylamide,
N-[6-(1-benzoyl-piperidin-4-yl)-hexyl]-3-(pyridin-3-yl)-acrylamide,
N-[4-(1-benzoyl-piperidin-4-yl)-butyl]-5-(pyridin-3-yl)-2,4-pentadienoic acid amide,
N-[4-(4-benzoyl-piperidin-1-yl)-butyl]-3-(pyridin-3-yl)-acrylamide,
N-[4-(4-benzoyl-piperidin-1-yl)-butyl]-3-(pyridin-3-yl)-propionamide,
N-[4-(1-benzylpiperidin-4-yl)-butyl]-3-(pyridin-3-yl)-propionamide,
N-[4-(1-diphenylmethyl-piperidin-4-yl)-butyl]-3-(2-fluoropyridin-3-yl)-propionamide,
N-[4-(1-diphenylmethyl-piperidin-4-yl)-butyl]-3-(5-fluoropyridin-3-yl)-propionamide,
N-[4-(1-diphenylmethyl-piperidin-4-yl)-butyl]-2-fluoro-3-(pyridin-3-yl)-propionamide,
N-[4-(1-diphenylmethyl-piperidin-4-yl)-butyl]-2,2-difluoro-3-(pyridin-3-yl)-propionamide,
N-[5-(1-diphenylmethyl-piperidin-4-yl)-pentyl]-3-(pyridin-3-yl)-propionamide,
N-[6-(1-diphenylmethyl-piperidin-4-yl)-hexyl]-3-(pyridin-3-yl)-propionamide,
N-[2-(1-diphenylmethyl-piperidin-4-yl)-ethyl]-5-(pyridin-3-yl)-pentanoic acid amide,
N-[4-(1-diphenylmethyl-piperidin-4-yl)-butyl]-3-(pyridin-3-yl)-propionamide,
N-[4-(1-diphenylmethyl-piperidin-4-yl)-butyl]-5-(pyridin-3-yl)-pentanoic acid amide,
N-[2-(1-diphenylmethylpiperidin-4-yl)-ethyl]-5-(pyridin-3-yl)-2,4-pentadienoic acid amide,
N-[4-(1-diphenylmethylpiperidin-4-yl)-butyl]-5-(pyridin-3-yl)-2,4-pentadienoic acid amide,
N-[5-(1-diphenylmethylpiperidin-4-yl)-pentyl]-5-(pyridin-3-yl)-2,4-pentadienoic acid amide,
N-[6-(1-diphenylmethylpiperidin-4-yl)-hexyl]-5-(pyridin-3-yl)-2,4-pentadienoic acid amide,
N-[4-(4-phenyl-piperidin-1-yl)-butyl]-3-pyridin-3-yl-acrylamide,
N-[4-(4,4-diphenyl-piperidin-1-yl)-butyl]-3-pyridin-3-yl-acrylamide,
N-[4-(1-benzoyl-2,6-dioxo-piperidin-4-yl)-butyl]-3-(pyridin-3-yl)-acrylamide,
N-[4-(2,6-dioxo-3-phenyl-piperidin-1-yl)-butyl]-3-pyridin-3-yl-acrylamide,
N-[4-(2,6-dioxo-4-phenyl-piperidin-1-yl)-butyl]-3-pyridin-3-yl-acrylamide,
N-[4-(4-benzoyl-piperazin-1-yl)-butyl]-3-(pyridin-3-yl)-acrylamide,
N-[4-(4-benzoyl-piperazin-1-yl)-butyl]-3-(pyridin-3-yl)-propionamide,
N-[4-(4-diphenylacetyl-piperazin-1-yl)-butyl]-3-pyridin-3-yl-acrylamide,
N-[4-(4-diphenylmethyl-piperazin-1-yl)-butyl]-3-pyridin-3-yl-propionamide,
N-[5-(4-diphenylmethyl-piperazin-1-yl)-pentyl]-3-pyridin-3-yl-acrylamide,
N-[6-(4-diphenylmethyl-piperazin-1-yl)-hexyl]-3-pyridin-3-yl-acrylamide,
N-[4-(4-diphenylmethyl-piperazin-1-yl)-butyl]-2-(pyridin-3-yl)-propionamide,
N-[4-(4-diphenylmethyl-piperazin-1-yl)-butyl]-5-(pyridin-3-yl)-penta-2,4-dienoic acid amide, and
N-[4-(4-benzyl-2,6-dioxo-piperazin-1-yl)-butyl]-3-pyridin-3-yl-acrylamide.

9. Use of a compound of Formula I or a pharmaceutically acceptable salt thereof, as defined according to any one of claims 1 to 8, for the manufacture of a pharmaceutical composition for the prevention or treatment of a disease or medical condition in a mammal, which disease or medical condition responds to inhibition or reduction of VEGF production and is selected from rheumatoid arthritis; inflammatory disorder; macular degeneration, especially age-related macular degeneration; retinopathy, especially proliferative retinopathy and diabetic retinopathy; and hyperplasia, especially benign prostatic hyperplasia and venous neointimal hyperplasia.

10. A compound of Formula I or a pharmaceutically acceptable salt thereof, as defined according to any one of claims 1 to 8, for use in the prevention or treatment of a disease or medical condition in a mammal, which disease or medical condition responds to inhibition or reduction of angiogenesis and is selected from: rheumatoid arthritis; inflammatory disorder; macular degeneration, especially age-related macular degeneration; retinopathy, especially proliferative retinopathy and diabetic retinopathy; and hyperplasia, especially benign prostatic hyperplasia and venous neointimal hyperplasia, by inhibition or reduction of angiogenesis in a mammal.

11. A compound of Formula I or a pharmaceutically acceptable salt thereof, as defined according to any one of claims 1 to 8, for use in the prevention or treatment of a disease or medical condition in a mammal, which disease or medical condition responds to VEGF production and is selected from rheumatoid arthritis; inflammatory disorder; macular degeneration, especially age-related macular degeneration; retinopathy, especially proliferative retinopathy and diabetic retinopathy; and hyperplasia, especially benign prostatic hyperplasia and venous neointimal hyperplasia.

## Patentansprüche

1. Verwendung einer Verbindung der Formel (I) oder eines pharmazeutisch akzeptablen Salzes davon zur Herstellung einer pharmazeutischen Zusammensetzung zur Vorbeugung oder Behandlung einer Krankheit oder von Beschwerden bei einem Säuger, wobei die Krankheit oder die Beschwerden auf die Inhibierung oder Reduzierung der Angiogenese anspricht und aus Gelenkrheumatismus; einer entzündlichen Erkrankung; Makuladegeneration, insbesondere altersbedingte Makuladegeneration; Retinopathie, insbesondere proliferative Retinopathie und diabetische Retinopathie; und Hyperplasie, insbesondere benigne Prostatahyperplasie und venöse neointimale Hyperplasie, ausgewählt wird: 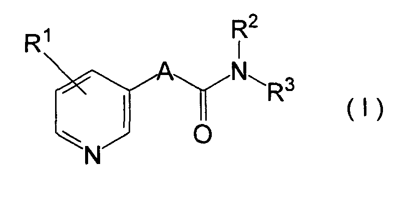 worin:
A aus der Gruppe, bestehend aus den Gruppenmitgliedern C₁₋₁₀-Alkylen, C₂₋₁₀-Alkenylen und
C₂₋₁₀-Alkinylen, ausgewählt wird, wobei die Gruppenmitglieder gegebenenfalls mit einer, zwei oder drei Gruppen, unabhängig voneinander ausgewählt aus C₁₋₃-Alkyl, Fluor, Chlor und Brom, substituiert sein können;
R¹ aus Wasserstoff, C₁₋₆-Alkyl, Fluor, Chlor, Brom und Perfluor-C₁₋₃-alkyl ausgewählt wird;
R² aus Wasserstoff, C₁₋₆-Alkyl und C₂₋₆-Alkenyl ausgewählt wird;
R³ aus der Gruppe ausgewählt wird, die aus den Gruppenmitgliedern C₁₋₆-Alkyl, (C₅₋₈-Cycloalkyl)-C₁₋₆-alkyl, (C₅₋₈-Heterocyclyl)-C₁₋₆-alkyl, C₁₋₆-Alkyl-(C₅₋₈-heterocyclyl)-C₁₋₆-alkyl und C₁₋₅-Alkylcarbonyl-(C₅₋₈-heterocyclyl)-C₁₋₆-alkyl besteht, wobei die Gruppenmitglieder gegebenenfalls mit einer, zwei oder drei Gruppen, unabhängig voneinander ausgewählt aus C₁₋₆-Alkyl, Fluor, Chlor, Brom, Oxo, Perfluor-C₁₋₃-alkyl, Aryl, Arylcarbonyl, Heteroaryl, Heteroarylcarbonyl, C₅₋₈-Cycloalkyl und C₅₋₈-Heterocyclyl, substituiert sein können.

2. Verwendung gemäss Anspruch 1, worin A aus Ethylen, n-Propylen, i-Propylen, n-Butylen, Ethenylen, 1-Propenylen, 1-Butenylen, 2-Butenylen und Ethinylen ausgewählt wird.

3. Verwendung gemäss Anspruch 1 oder 2, worin R¹ aus Wasserstoff, Methyl, Ethyl, n-Propyl, Fluor und Trifluormethyl ausgewählt wird.

4. Verwendung gemäss mindestens einem der Ansprüche 1 bis 3, worin R² aus Wasserstoff, Methyl, Ethyl, n-Propyl und Ethenyl ausgewählt wird.

5. Verwendung gemäss mindestens einem der Ansprüche 1 bis 4, worin R³ aus der Gruppe ausgewählt wird, die aus den Gruppenmitgliedern Cyclopentyl-C₁₋₆-alkyl, Cyclohexyl-C₁₋₆-alkyl, Pyrrolidinyl-C₁₋₆-alkyl, Piperidinyl-C₁₋₆-alkyl, C₁₋₆-Alkyl-piperidinyl-C₁₋₆-alkyl, C₁₋₅-Alkylcarbonyl-piperidinyl-C₁₋₆-alkyl, Piperazinyl-C₁₋₆-alkyl, C₁₋₆-Alkyl-piperazinyl-C₁₋₆-alkyl, C₁₋₅-Alkylcarbonyl-piperazinyl-C₁₋₆-alkyl und Morpholinyl-C₁₋₆-alkyl besteht, wobei die Mitglieder mit einer, zwei oder drei Gruppen, ausgewählt aus C₁₋₆-Alkyl, Fluor, Chlor, Brom, Oxo, Perfluor-C₁₋₃-alkyl, Aryl, Arylcarbonyl, Heteroaryl, C₅₋₈-Cycloalkyl und C₅₋₈-Heterocyclyl, substituiert sein können.

6. Verwendung gemäss mindestens einem der Ansprüche 1 bis 5, worin R³ aus der Gruppe ausgewählt wird, die aus den folgenden Gruppenmitgliedern besteht: Cyclohexyl-C₁₋₆-alkyl, Piperidinyl-C₁₋₆-alkyl, C₁₋₆-Alkyl-piperidinyl-C₁₋₆-alkyl, C₁₋₅-Alkylcarbonyl-piperidinyl-C₁₋₆-alkyl, Piperazinyl-C₁₋₆-alkyl, C₁₋₆-Alkyl-piperazinyl-C₁₋₆-alkyl, C₁₋₅-Alkylcarbonyl-piperazinyl-C₁₋₆-alkyl, wobei die Mitglieder gegebenenfalls mit einer, zwei oder drei Gruppen, unabhängig voneinander ausgewählt aus Butyl, Pentyl, Hexyl, Fluor, Oxo, Phenyl, Biphenyl, Benzyl, Pyridyl, Pyrrolyl, Benzoyl, Thiophenyl, Furyl, Cyclopentyl, Cyclohexyl und Piperidinyl, substituiert sein können.

7. Verwendung gemäss mindestens einem der Ansprüche 1 bis 6, worin R³ aus der Gruppe ausgewählt wird, die aus
(1-Acetyl-piperidin-4-yl)-butyl,
(1-Diphenylacetyl-piperidin-4-yl)-butyl,
[1-(3,3-Diphenylpropionyl)-piperidin-4-yl]-butyl,
(1-Benzoyl-piperidin-4-yl)-ethyl,
(1-Benzoyl-piperidin-4-yl)-propyl,
(1-Benzoyl-piperidin-4-yl)-butyl,
(1-Benzoyl.-piperidin-4-yl)-pentyl,
(1-Benzoyl-piperidin-4-yl)-hexyl,
(1-Benzoyl-piperidin-4-yl)-butyl,
(1-Diphenylmethyl-piperidin-4-yl)-methyl, (1-Diphenylmethyl-piperidin-4-yl)-ethyl,
(1-Diphenylmethyl-piperidin-4-yl)-propyl,
(1-Diphenylmethyl-piperidin-4-yl)-butyl,
(1-Diphenylmethyl-piperidin-4-yl)-pentyl,
(1-Diphenylmethyl-piperidin-4-yl)-hexyl,
(4-Phenyl-piperidin-1-yl)-butyl,
(4,4-Diphenyl-piperidin-1-yl)-butyl,
(1-Benzoyl-2,6-dioxo-piperidin-4-yl)-butyl,
(2,6-Dioxo-3-phenyl-piperidin-1-yl)-butyl,
(2,6-Dioxo-4-phenyl-piperidin-1-yl)-butyl,
(4-Phenyl-piperazin-1-yl)-butyl,
(4-Phenyl-piperazin-1-yl)-pentyl,
(4-Phenyl-piperazin-1-yl)-hexyl,
(4-Diphenylacetyl-piperazin-1-yl)-butyl,
(4-Benzoylpiperazin-1-yl)-butyl und
(4-Benzyl-2,6-dioxo-piperazin-1-yl)-butyl
besteht.

8. Verwendung gemäss mindestens einem der Ansprüche 1 bis 7, worin die Verbindung der Formel (I) aus der Gruppe ausgewählt wird, die aus
N-[4-(1-Acetyl-piperidin-4-yl)-butyl]-3-(pyridin-3-yl)-propionamid,
N-[4-(1-Acetyl-piperidin-4-yl)-butyl]-3-(pyridin-3-yl)-acrylamid,
N-[4-(1-Diphenylacetyl-piperidin-4-yl)-butyl]-3-(pyridin-3-yl)-acrylamid,
N-[4-(1-Diphenylacetyl-piperidin-4-yl)-butyl]-3-(pyridin-3-yl)-propionamid,
N-{4-[1-(3,3-Diphenylpropionyl)-piperidin-4-yl]-butyl}-3-(pyridin-3-yl)-acrylamid, N-[3-(1-Benzoyl-piperidin-4-yl)-propyl]-3-(pyridin-3-yl)-propionamid,
N-[4-(1-Benzoyl-piperidin-4-yl)-butyl]-3-(pyridin-3-yl)-propionamid,
N-[6-(1-Benzoyl-piperidin-4-yl)-hexyl]-3-(pyridin-3-yl)-propionamid,
N-[2-(1-Benzoyl-piperidin-4-yl)-ethyl]-3-(pyridin-3-yl)-acrylamid,
N-[4-(1-Benzoyl-piperidin-4-yl)-butyl]-3-(pyridin-3-yl)-acrylamid,
N-[6-(1-Benzoyl-piperidin-4-yl)-hexyl]-3-(pyridin-3-yl)-acrylamid,
N-[4-(1-Benzoyl-piperidin-4-yl)-butyl]-5-(pyridin-3-yl)-2,4-pentadiensäureamid,
N-[4-(4-Benzoyl-piperidin-1-yl)-butyl]-3-(pyridin-3-yl)-acrylamid,
N-[4-(4-Benzoyl-piperidin-1-yl)-butyl]-3-(pyridin-3-yl)-propionamid,
N-[4-(1-Benzylpiperidin-4-yl)-butyl]-3-(pyridin-3-yl)-propionamid,
N-[4-(1-Diphenylmethyl-piperidin-4-yl)-butyl]-3-(2-fluorpyridin-3-yl)-propionamid,
N-[4-(1-Diphenylmethyl-piperidin-4-yl)-butyl]-3-(5-fluorpyridin-3-yl)-propionamid,
N-[4-(1-Diphenylmethyl-piperidin-4-yl)-butyl]-2-fluor-3-(pyridin-3-yl)-propionamid,
N-[4-(1-Diphenylmethyl-piperidin-4-yl)-butyl]-2,2-difluor-3-(pyridin-3-yl)-propionamid,
N-[5-(1-Diphenylmethyl-piperidin-4-yl)-pentyl]-3-(pyridin-3-yl)-propionamid,
N-[6-(1-Diphenylmethyl-piperidin-4-yl)-hexyl]-3-(pyridin-3-yl)-propionamid,
N-[2-(1-Diphenylmethyl-piperidin-4-yl)-ethyl]-5-(pyridin-3-yl)-pentansäureamid,
N-[4-(1-Diphenylmethyl-piperidin-4-yl)-butyl]-3-(pyridin-3-yl)-propionamid,
N-[4-(1-Diphenylmethyl-piperidin-4-yl)-butyl]-5-(pyridin-3-yl)-pentansäureamid,
N-[2-(1-Diphenylmethyl-piperidin-4-yl)-ethyl]-5-(pyridin-3-yl)-pentadiensäureamid, N-[4-(1-Diphenylmethyl-piperidin-4-yl)-butyl]-5-(pyridin-3-yl)-pentadiensäureamid,
N-[5-(1-Diphenylmethyl-piperidin-4-yl)-pentyl]-5-(pyridin-3-yl)-2,4-pentadiensäureamid,
N-[6-(1-Diphenylmethyl-piperidin-4-yl)-hexyl]-5-(pyridin-3-yl)-2,4-pentadiensäureamid,
N-[4-(4-Phenyl-piperidin-1-yl)-butyl]-3-(pyridin-3-yl)-acrylamid,
N-[4-(4,4-Diphenyl-piperidin-1-yl)-butyl]-3-pyridin-3-yl-acrylamid,
N-[4-(1-Benzoyl-2,6-dioxo-piperidin-4-yl)-butyl-3-(pyridin-3-yl)-acrylamid,
N-[4-(2,6-Dioxo-3-phenyl-piperidin-1-yl)-butyl]-3-pyridin-3-yl-acrylamid,
N-[4-(2,6-Dioxo-4-phenyl-piperidin-1-yl)-butyl]-3-pyridin-3-yl-acrylamid,
N-[4-(4-Benzoyl-piperazin-1-yl)-butyl]-3-(pyridin-3-yl)-acrylamid,
N-[4-(4-Benzoyl-piperazin-1-yl)-butyl-3]-(pyridin-3-yl)-propionamid,
N-[4-(4-Diphenylacetyl-piperazin-1-yl)-butyl]-3-pyridin-3-yl-acrylamid,
N-[4-(4-Diphenylmethyl-piperazin-1-yl)-butyl]-3-pyridin-3-yl-propionamid,
N-[5-(4-Diphenylmethyl-piperazin-1-yl)-pentyl]-3-pyridin-3-yl-acrylamid,
N-[6-(4-Diphenylmethyl-piperazin-1-yl)-hexyl]-3-pyridin-3-yl-acrylamid,
N-[4-(4-Diphenylmethyl-piperazin-1-yl)-butyl]-2-(pyridin-3-yl)-propionamid,
N-[4-(4-Diphenylmethyl-piperazin-1-yl)-butyl]-5-(pyridin-3-yl)-penta-2,4-diensäureamid und
N-[4-(4-Benzyl-2,6-dioxo-piperazin-1-yl)-butyl]-3-pyridin-3-yl-acrylamid
besteht.

9. Verwendung einer Verbindung gemäss Formel (I) oder eines pharmazeutisch akzeptablen Salzes davon, wie in mindestens einem der Ansprüche 1 bis 8 definiert, zur Herstellung einer pharmazeutischen Zusammensetzung zur Vorbeugung oder Behandlung einer Krankheit oder von Beschwerden bei einem Säuger, wobei die Krankheit oder die Beschwerden auf die Inhibierung oder Reduzierung der VEGF-Produktion anspricht und aus Gelenkrheumatismus; einer entzündlichen Erkrankung; Makuladegeneration, insbesondere altersbedingte Makuladegeneration; Retinopathie, insbesondere proliferative Retinopathie und diabetische Retinopathie; und Hyperplasie, insbesondere benigne Prostatahyperplasie und venöse neointimale Hyperplasie, ausgewählt wird.

10. Verbindung der Formel (I) oder eines pharmazeutisch akzeptablen Salzes davon, wie in mindestens einem der Ansprüche 1 bis 8 definiert, zur Verwendung in der Vorbeugung oder Behandlung einer Krankheit oder von Beschwerden bei einem Säuger, wobei die Krankheit oder die Beschwerden auf die Inhibierung oder Reduzierung der Angiogenese anspricht und aus folgenden ausgewählt wird: Gelenkrheumatismus; einer entzündlichen Erkrankung; Makuladegeneration, insbesondere altersbedingte Makuladegeneration; Retinopathie, insbesondere proliferative Retinopathie und diabetische Retinopathie; und Hyperplasie, insbesondere benigne Prostatahyperplasie und venöse neointimale Hyperplasie, durch Inhibierung oder Reduzierung der Angiogenese in einem Säuger.

11. Verbindung der Formel (I) oder ein pharmazeutisch akzeptables Salz davon, wie in mindestens einem der Ansprüche 1 bis 8 definiert, zur Verwendung in der Vorbeugung oder Behandlung einer Krankheit oder von Beschwerden bei einem Säuger, wobei die Krankheit oder die Beschwerden auf die VEGF-Produktion anspricht und aus Gelenkrheumatismus; einer entzündlichen Erkrankung; Makuladegeneration, insbesondere altersbedingte Makuladegeneration; Retinopathie, insbesondere proliferative Retinopathie und diabetische Retinopathie; und Hyperplasie, insbesondere benigne Prostatahyperplasie und venöse neointimale Hyperplasie, ausgewählt wird.

## Revendications

1. Utilisation d'un composé de formule I ou d'un sel pharmaceutiquement acceptable de celui-ci, pour la préparation d'une composition pharmaceutique pour la prévention ou le traitement d'une maladie ou d'un état médical chez un mammifère, laquelle maladie ou état médical répond à l'inhibition ou la réduction de l'angiogenèse et est choisie parmi la polyarthrite rhumatoïde; un désordre inflammatoire ; la dégénérescence maculaire, en particulier la dégénérescence maculaire liée à l'âge ; la rétinopathie, en particulier la rétinopathie proliférative et la rétinopathie diabétique, et l'hyperplasie, en particulier une hyperplasie prostatique bénigne et l'hyperplasie néointimale veineuse : 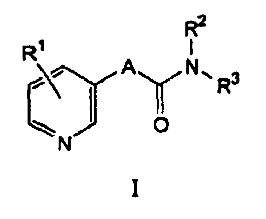 dans laquelle :
A est choisi parmi le groupe consistant en les éléments alkylène en C₁₋₁₀, alcénylène en C₂₋₁₀ et alcynylène en C₂₋₁₀, lesquels éléments peuvent être le cas échéant substitués par un, deux ou trois éléments indépendamment choisis parmi alkyle en C₁₋₃, fluoro, chloro et bromo ;
R¹ est choisi parmi hydrogène, alkyle en C₁₋₆, fluoro, chloro, bromo et perfluoroalkyle en C₁₋₃ ;
R² est choisi parmi hydrogène, alkyle en C₁₋₆ et alcényle en C₂₋₆;
R³ est choisi parmi le groupe consistant en les éléments alkyle en C₁₋₆, (cycloalkyle en C₅₋₈)-alkyle en C₁₋₆, (hétérocyclyl en C₅₋₈)-alkyle en C₁₋₆, alkyle en C₁₋₆-(hétérocyclyl en C₅₋₈)-alkyle en C₁₋₆, et (alkyle en C₁₋₅)carbonyl(hétérocyclyl en C₅-₈)-alkyle en C₁₋₆, lesquels éléments peuvent être éventuellement substitués par un, deux ou trois groupes indépendamment choisis parmi alkyle en C₁₋₆, fluoro, chloro, bromo, oxo, perfluoroalkyle en C₁₋₃, aryle, arylcarbonyle, hétéroaryle, hétéroarylcarbonyle, cycloalkyle en C₅₋₈ et hétérocyclyle en C₅₋₈.

2. Utilisation selon la revendication 1, dans laquelle A est choisi parmi l'éthylène, le n-propylène, l'isopropylène, le n-butylène, l'éthénylène, le 1-propénylène, le 1-buténylène, le 2-buténylène et l'éthynylène.

3. Utilisation selon la revendication 1 ou 2, dans laquelle R¹ est choisi parmi hydrogène, méthyle, éthyle, n-propyle, fluoro et trifluorométhyle.

4. Utilisation selon l'une quelconque des revendications 1 à 3, dans laquelle R² est choisi parmi hydrogène, méthyle, éthyle, n-propyle et éthényle.

5. Utilisation selon l'une quelconque des revendications 1 à 4, dans laquelle R³ est choisi parmi le groupe consistant en les éléments cyclopentyl-alkyle en C₁₋₆, cyclohexyl-alkyle en C₁₋₆, pyrrolidinyl-alkyle en C₁₋₆, pipéridinyl-alkyle en C₁₋₆, (alkyle en C₁₋₆)pipéridinyl-alkyle en C₁₋₆, (alkyle en C₁₋₅)carbonylpipéridinyl-alkyle en C₁₋₆, pipérazinyl-alkyle en C₁₋₆, (alkyle en C₁₋₆)pipérazinyl-alkyle en C₁₋₆, (alkyl en C₁₋₅)carbonylpipérazinyl-alkyle en C₁₋₆, et morpholinyl-alkyle en C₁₋₆, lesquels éléments peuvent être éventuellement substitués par un, deux ou trois éléments indépendamment choisis parmi alkyle en C₁₋₆, fluoro, chloro, bromo, oxo, perfluoroalkyle en C₁₋₃, aryle, arylcarbonyle, hétéroaryle, cycloalkyle en C₅₋₈ et hétérocyclyle en C₅₋₈.

6. Utilisation selon l'une quelconque des revendications 1 à 5, dans laquelle R³ est choisi parmi le groupe consistant en les éléments cyclohexyl-alkyle en C₁₋₆, pipéridinyl-alkyle en C₁₋₆, (alkyle en C₁₋₆)pipéridinyl-alkyle en C₁₋₆, (alkyle en C₁₋₅)carbonylpipéridinyl-alkyle en C₁₋₆, pipérazinyl-alkyle en C₁₋₆, (alkyle en C₁₋₆)pipérazinyl-alkyle en C₁₋₆, (alkyl en C₁₋₅)carbonylpipérazinyl -alkyle en C₁₋₆, lesquels éléments peuvent être éventuellement substitués par un, deux ou trois éléments indépendamment choisis parmi butyle, pentyle, hexyle, fluoro, oxo, phényle, biphényle, benzyle, pyridyle, pyrrolyle, benzoyle, thiényle, furyle, cyclopentyle, cyclohexyle et pipéridinyle.

7. Utilisation selon l'une quelconque des revendications 1 à 6, dans laquelle R³ est choisi parmi le groupe consistant en
(1-acétylpipéridin-4-yl)butyle,
(1-diphénylacétylpipéridin-4-yl)butyle,
[1-(3,3-diphénylpropionyl)pipéridin-4-yl]butyle,
(1-benzoylpipéridin-4-yl)éthyle,
(1-benzoylpipéridin-4-yl)propyle,
(1-benzoylpipéridin-4-yl)butyle,
(1-benzoylpipéridin-4-yl)pentyle,
(1-benzoylpipéridin-4-yl)hexyle,
(1-benzylpipéridin-4-yl)butyle,
(1-diphénylméthylpipéridin-4-yl)méthyle,
(1-diphénylméthylpipéridin-4-yl)éthyle,
(1-diphénylméthylpipéridin-4-yl)propyle,
(1-diphénylméthylpipéridin-4-yl)butyle,
(1-diphénylméthylpipéridin-4-yl)pentyle,
(1-diphénylméthylpipéridin-4-yl)hexyle,
(4-phénylpipéridin-1 -yl)butyle,
(4,4-diphénylpipéridin- 1 -yl)butyle,
(1-benzoyl-2,6-dioxopipéridin-4-yl)butyle,
(2,6-dioxo-3-phénylpipéridin-1-yl)butyle,
(2,6-dioxo-4-phénylpipéridin-1-yl)butyle,
(4-phénylpipérazin- 1 -yl)butyle,
(4-phénylpipérazin- 1 -yl)pentyle,
(4-phénylpipérazin- 1 -yl)hexyl e,
(4-diphénylacétylpipérazin- 1 -yl)butyle,
(4-benzoylpipérazin-1-yl)butyle, et
(4-benzyl-2,6-dioxopipérazin-1-yl)butyle.

8. Utilisation selon l'une quelconque des revendications 1 à 7, dans laquelle le composé de formule 1 est choisi parmi le groupe consistant en
le N-[4-(1-acétylpipéridin-4-yl)butyl]-3-(pyridin-3-yl)propionamide,
le N-[4-(1-acétylpipéridin-4-yl)butyl]-3-(pyridin-3-yl)acrylamide,
le N-[4-(1-diphénylacétylpipéridin-4-yl)butyl]-3-(pyridin-3-yl)acrylamide,
le N-[4-(1-diphénylacétylpipéridin-4-yl)butyl]-3-(pyridin-3-yl)propionamide,
le N-[4-[1-(3,3-diphénylpropionyl)pipéridin-4-yl]butyl}-3-(pyridin-3-yl)acrylamide,
le N-[3-(1-benzoylpipéridin-4-yl)propyl]-3-(pyridin-3-yl)propionamide,
le N-[4-(1 -benzoylpipéridin-4-yl)butyl]-3-(pyridin-3-yl)propionamide,
le N-[6-(-benzoylpipéridin-4-yl)hexyl] -3 -(pyridin-3 -yl)propionamide,
le N-[2-(1-benzoylpipéridin-4-yl)éthyl]-3-(pyridin-3-yl)acrylamide,
le N-[4-(1-benzoylpipéridin-4-yl)butyl]-3-(pyridin-3-yl)acrylamide,
le N-[6-(-benzoylpipéridin-4-yl)hexyl] -3 -(pyridin-3 -yl)acrylamide,
le N-[4-(1-benzoylpipéridin-4-yl)butyl]amide de l'acide 5-(pyridin-3-yl)-2,4-pentadiènoïque,
le N-[4-(4-benzoylpipéridin-1-yl)butyl]-3-(pyridin-3-yl)acrylamide,
le N-[4-(4-benzoylpipéridin-1-yl)butyl]-3-(pyridin-3-yl)propionamide,
le N-[4-(1-benzylpipéridin-1-yl)butyl]-3-(pyridin-3-yl)propionamide,
le N-[4-(1-diphénylméthylpipéridin-4-yl)butyl]-3-(2-fluoropyridin-3-yl)-propionamide,
le N-[4-(1-diphénylméthylpipéridin-4-yl)butyl]-3-(5-fluoropyridin-3-yl)-propionamide,
le N-[4-(1-diphénylmétllylpipéridin-4-yl)butyl]-2-fluoro-3-(pyridin-3-yl)-propionamide,
le N-[4-(1-diphénylméthylpipéridin-4-yl)butyl]-2,2-difluoro-3-(pyridin-3-yl)-propionamide,
le N-[5-(1-diphénylméthylpipéridin-4-yl)pentyl]-3-(pyridin-3-yl)propionamide,
le N-[6-(1-diphénylméthylpipéridin-4-yl)hexyl]-3-(pyridin-3-yl)propionamide,
le N-[2-(1-diphénylméthylpipéridin-4-yl)ethyl]amide de l'acide 5-(pyridin-3-yl)-pentanoïque,
le N-[4-(1-diphénylméthylpipéridin-4-yl)butyl]-3-(pyridin-3-yl)propionamide,
le N-[4-(1-diphénylméthylpipéridin-4-yl)butyl]amide de l'acide 5-(pyridin-3-yl)-pentanoïque,
le N-[2-(1-diphénylméthylpipéridin-4-yl)ethyl]amide de l'acide 5-(pyridin-3-yl)-2,4-pentadiènoïque,
le N-[4-(1-diphénylméthylpipéridin-4-yl)butyl]amide de l'acide 5-(pyridin-3-yl)-2,4-pentadiènoïque,
le N-[5-(1-diphénylméthylpipéridin-4-yl)pentyl]amide de l'acide 5-(pyridin-3-yl)-2,4-pentadiènoïque,
le N-[6-(1-diphénylméthylpipéridin-4-yl)hexyl]amide de l'acide 5-(pyridin-3-yl)-2,4-pentadiènoïque,
le N-[4-(4-phénylpipéridin-1-yl)butyl]-3-(pyridin-3-yl)acrylamide,
le N-[4-(4,4-diphénylpipéridin-1-yl)butyl]-3-(pyridin-3-yl)acrylamide
le N-[4-(1-benzoyl-2,6-dioxopipéridin-4-yl)butyl]-3-(pyridin-3-yl)acrylamide,
le N-[4-(2,6-dioxo-3-phénylpipéridin-1-yl)butyl]-3-(pyridin-3-yl)acrylamide,
le N-[4-(2,6-dioxo-4-phénylpipéridin-1-yl)butyl]-3-(pyridin-3-yl)acrylamide,
le N-[4-(4-benzoylpipérazin-1-yl)butyl]-3-(pyridin-3-yl)acrylamide,
le N-[4-(4-benzoylpipérazin-1-yl)butyl]-3-(pyridin-3-yl)propionamide,
le N-[4-(4-diphénylacétylpipérazin-1-yl)butyl]-3-(pyridin-3-yl)acrylamide,
le N-[4-(4-diphénylméthylpipérazin-1-yl)butyl]-3-(pyridin-3-yl)propionamide,
le N-[5-(4-diphénylméthylpipérazin- 1 -yl)pentyl]-3 -(pyridin-3-yl)acrylamide,
le N-[6-(4-diphénylméthylpipérazin-1-yl)hexyl]-3-(pyridin-3-yl)acrylamide,
le N-[4-(4-diphénylméthylpipérazin-1-yl)butyl]-2-(pyridin-3-yl)propionamide,
le N-[4-(4-diphénylméthylpipérazin-1-yl)butyl]amide de l'acide 5-(pyridin-3-yl)-2,4-pentadiènoïque, et
le N-[4-(4-benzyl-2,6-dioxopipérazin-1-yl)butyl]-3-(pyridin-3-yl)acrylamide.

9. Utilisation d'un composé de formule I ou d'un sel pharmaceutiquement acceptable de celui-ci, tel que défini selon l'une quelconque des revendications 1 à 8, pour la préparation d'une composition pharmaceutique pour la prévention ou le traitement d'une maladie ou d'un état médical chez un mammifère, laquelle maladie ou état médical répond à l'inhibition ou la réduction de la production de VEGF et est choisie parmi la polyarthrite rhumatoïde ; un désordre inflammatoire ; la dégénérescence maculaire, en particulier la dégénérescence maculaire liée à l'âge ; la rétinopathie, en particulier la rétinopathie proliférative et la rétinopathie diabétique, et l'hyperplasie, en particulier une hyperplasie prostatique bénigne et l'hyperplasie néointimale veineuse.

10. Composé de formule I ou un sel pharmaceutiquement acceptable de celui-ci, tel que défini selon l'une quelconque des revendications 1 à 8, à utiliser dans la prévention ou le traitement d'une maladie ou d'un état médical chez un mammifère, laquelle maladie ou état médical répond à l'inhibition ou la réduction de l'angiogenèse et est choisie parmi la polyarthrite rhumatoïde ; un désordre inflammatoire ; la dégénérescence maculaire, en particulier la dégénérescence maculaire liée à l'âge ; la rétinopathie, en particulier la rétinopathie proliférative et la rétinopathie diabétique, et l'hyperplasie, en particulier une hyperplasie prostatique bénigne et l'hyperplasie néointimale veineuse, par inhibition ou réduction de l'angiogenèse chez un mammifère.

11. Composé de formule I ou un sel pharmaceutiquement acceptable de celui-ci, tel que défini selon l'une quelconque des revendications 1 à 8, à utiliser dans la prévention ou le traitement d'une maladie ou d'un état médical chez un mammifère, laquelle maladie ou état médical répond à l'inhibition ou la réduction de la production de VEGF et est choisie parmi la polyarthrite rhumatoïde ; un désordre inflammatoire ; la dégénérescence maculaire, en particulier la dégénérescence maculaire liée à l'âge ; la rétinopathie, en particulier la rétinopathie proliférative et la rétinopathie diabétique, et l'hyperplasie, en particulier une hyperplasie prostatique bénigne et l'hyperplasie néointimale veineuse.
